(19) Europäisches Patentamt
European Patent Office
Office européen des brevets

(11) Publication number: **0 334 119 B1**

# (12) EUROPEAN PATENT SPECIFICATION

(45) Date of publication of patent specification: **16.06.93**

(51) Int. Cl.5: **C07D 333/16**, C07D 213/30, C07C 39/21, C07C 43/23, A61K 31/33, A61K 31/05

(21) Application number: **89104251.7**

(22) Date of filing: **10.03.89**

## (54) Compounds for inhibiting the biosynthesis of lipoxygenase-derived metabolites of arachidonic acid.

(30) Priority: **21.03.88 US 170512**

(43) Date of publication of application: **27.09.89 Bulletin 89/39**

(45) Publication of the grant of the patent: **16.06.93 Bulletin 93/24**

(84) Designated Contracting States: **AT BE CH DE ES FR GB GR IT LI LU NL SE**

(56) References cited:
**EP-A- 0 276 051**
**US-A- 3 188 315**
**US-A- 3 390 147**
**US-A- 4 743 606**

**CHEMICAL ABSTRACT, vol. 76, no. 25, June 19, 1972, Columbus, Ohio, USA, G. N. BOGDANOV et al. "Synthesis and properties of sterically hindered hydroxystyrylpyridines." page 445, column 1, abstract-no. 153 516s**

(73) Proprietor: **BOEHRINGER INGELHEIM PHARMACEUTICALS INC.**
**900 Ridgebury Road, P.O. Box 368**
**Ridgefield, Connecticut 06877-0368(US)**

(72) Inventor: **Lazer, Edward S.**
**280 Beacon Hill Road**
**Trumbull, Connecticut 06611(US)**

(74) Representative: **Laudien, Dieter et al**
**Boehringer Ingelheim International GmbH**
**ZA Patente Postfach 200**
**W-6507 Ingelheim am Rhein- (DE)**

EP 0 334 119 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**CHEMICAL ABSTRACTS, vol. 75, no. 3, July 19, 1971, Columbus, Ohio, USA, M. FETIZON et al.: "Oxidations with silver carbonate/celite. V. Oxidations of phenols and related compounds.", page 416, column 1, abstracts-no. 19 855c**

**CHEMICAL ABSTRACTS, vol. 71, no. 5, August 4, 1969, Columbus, Ohio, USA, H.D. BECKER "New stable phenoxy radicals. Oxidation of hydroxystilbenes." page 291, column 2, abstracts-no. 21 813y**

**CHEMICAL ABSTRACTS, vol. 72, no. 13, March 30, 1970, Columbus, Ohio, USA, R.H. SIEBER "Reactions of chloroacetaldehyde with aromatic hydrocarbosn, phenols, and phenol ether.", page 349, column 1, abstract-no. 66 510j**

**CHEMICAL ABSTRACTS, vol. 92, no. 9, March 3, 1980, Columbus, Ohio, USA, J. GIERER et al. "Studies on the condensation of lignins in alkaline media. Part III. The formation of stilbenes, arylcoumarans and diarylmethanes on treatment of spruce wood meal with alkali and white liquor in the presence of xylenols.", page 105, column 2, abstract-no. 78 366t**

## Description

This invention relates to compounds, compositions and methods useful in treating symptoms of immunological and non-immunological disorders such as allergy, inflammation, shock or other disorders wherein metabolites of arachidonic acid are implicated.

The antigenic challenge of sensitized tissue, or damage induced in normal tissue through inflammatory insults or trauma, results in a broad array of tissue responses, including the production of various chemical mediators. One such mediator is slow reactive substance of anaphylaxis (SRS-A), the main components of which are the leukotrienes $LTC_4$, $LTD_4$ in humans and additionally $LTE_4$ in certain other species. Other important mediators are $LTB_4$ and other hydroxy and polyhydroxy metabolites of arachidonic acid which have been identified as being implicated in inflammatory processes.

The synthesis of these mediators in tissue initially requires the production of the biological precursors, arachidonic acid, as by action of phospholipase on cell membrane phospholipids. Subsequent metabolism of arachidonic acid by the lipoxygenase enzymes yields the unstable hydroperoxyeicosatetraenoic acids (HPETE's), subsequently the hydroxyeicosatetraenoic acids (HETE's), and various leukotrienes, including those which make up SRS-A. Arachidonic acid is also metabolized through the alternative cyclooxygenase pathway to yield the prostaglandins and the thromboxanes.

The primary pharmacological effects of SRS-A include smooth muscle contraction and increased vascular permeability. Its implication in human allergic asthma has been recognized for many years. More recently, SRS-A, $LTB_4$ and the (poly)hydroxymetabolites of arachidonic acid have been associated with shock, cardiovascular disease and ischemic tissue damage.

According to a broad aspect of the present invention we provide compounds having the general formula I

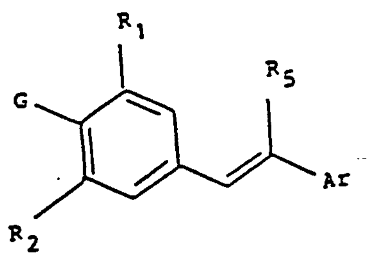

I

wherein

$R_1$ and $R_2$ are each independently branched or straight chained alkyl, allyl, alkoxy e.g. methoxy or halogen,

$R_5$ is hydrogen, alkyl or $-CO_2R_8$, wherein $R_8$ is hydrogen or $C_1$-$C_3$ alkyl

G is

a) -OH

b) the group $R_3CO_2$- wherein $R_3$ is (i) $HO_2C$-X wherein X is an optionally substituted hydrocarbon chain with or without heteroatoms, or (ii) the group

$$(R_{10})(R_{11})N\underset{R_{12}}{(CH)}_n$$

wherein $R_{10}$ and $R_{11}$ are hydrogen or alkyl, $R_{12}$ is hydrogen, alkyl or aryl possibly containing heteroatoms or $R_{11}$ and $R_{12}$ together form a ring, or

c) the group $HCO_2$- provided $R_5$ is $-CO_2R_8$ and $R_8$ is hydrogen or $C_1$-$C_3$ alkyl, and

Ar is an optionally substituted heteroaromatic or aromatic ring, with proviso that Ar is not

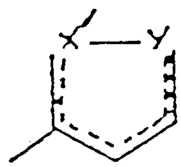

wherein --- is single or double bond and X' and Y are

(i) N

(ii) $NR_4$, where $R_4$ is H, lower alkyl,

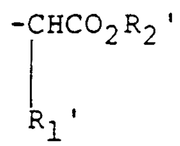

where $R_1$' and $R_2$' may be the same or different and are H or lower alkyl,

C(O)$R_4$' wherein $R_4$' is hydrogen and lower alkyl, cycloalkyl of from three to twenty carbons having from three to eight ring carbons, aryl or aralkyl;

(iii) O; or

(iv) S;

and the further proviso that Ar is not

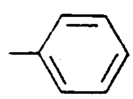
or
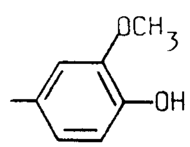

when $R_1$ and $R_2$ are both methyl,
and the further proviso that Ar is not p-pyridyl when G is hydroxy, $R_1$ is methyl, iso-propyl or tert. butyl and $R_2$ is iso-propyl, tert. butyl or cyclohexyl and $R_5$ hydrogen or methyl;
and the further proviso that Ar is not o-pyridyl when G is hydroxy, $R_2$ is methyl, iso-propyl or tert. butyl and $R_2$ is iso-propyl or tert. butyl, and $R_5$ is hydrogen;
or another pharmaceutically acceptable ester or pro-drug thereof or salt or acid addition salt of the foregoing compounds.

This invention thus relates inter alia to novel 4-(2-aryl-ethenyl)-2,6-disubstituted phenols of general formula 1':

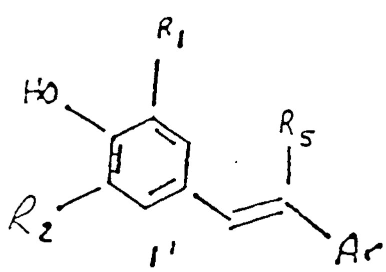

1'

wherein $R_1$ and $R_2$ are straight or branched chained alkyl e.g. $C_1$-$C_3$ alkyl, allyl, alkoxy or halogen, $R_5$ is hydrogen, alkyl, preferably $C_1$-$C_5$ alkyl or $CO_2R_8$, wherein $R^8$ is hydrogen or alkyl, and Ar is an optionally substituted heteroaromatic or aromatic ring.

This invention includes also the pharmacologically acceptable esters, pro-drugs, salts and acid addition salts of the compounds of formula 1'. For instance the invention also relates to esters (formulae 2a, 2b and

2c) of the compounds of formula 1', where the ester group imparts desirable physicochemical characteristics in the molecule, such as improved solubility. The esters are cleaved in vivo, releasing the compound 1' (i.e., they are pro-drugs).

In this specification unless otherwise specified the term "alkyl" means an alkyl group containing up to 8 carbon atoms e.g. up to 5 or 6 or 7 carbon atoms; and the term "lower alkyl" means an alkyl group containing 1 to 5 preferably 1 to 3 carbon atoms; "alkoxy" means an alkoxy group containing up to 8 carbon atoms preferably up to three carbon atoms and is especially methoxy; "halogen" means chlorine, bromine or fluorine preferably chlorine, bromine or fluorine; "heteroaromatic ring" means an aromatic group containing 5 to 12 e.g. up to 9 ring members of which up to 3 may be heteroatoms chosen from N, S or O and which may contain two fused rings; "aromatic ring" means an aromatic group containing five to 12 carbon atoms e.g. up to 10 carbon atoms and which may contain two fused rings; heteroatom includes nitrogen, sulphur or oxygen atoms; the substituents on the group Ar may be for example up to three groups which may be the same or different and chosen from $C_1$-$C_5$ alkoxy e.g. $C_1$-$C_3$ alkoxy, halogen, trifluoromethyl, alkyl and arylalkoxy.

The pro-drugs of the present invention can e.g. be of three types: monoesters of dicarboxylic acids (2a), esters of amino acids (2b), and substituted olefin esters or acids (2c), and also include salts of 2a, 2b and 2c, and alkyl or other esters of the acids of 2a and 2c.

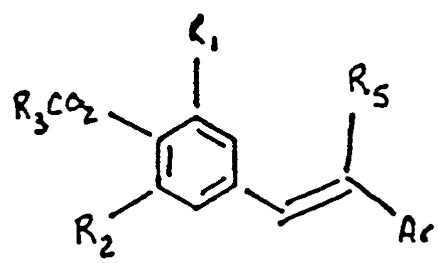

2a $R_1$, $R_2$, $R_5$, and Ar are as defined in Formula 1;
$R_3$ is $HO_2C$-X-, where X is an optionally substituted hydrocarbon chain with or without heteroatoms,

2b $R_1$, $R_2$, $R_5$, and Ar are as defined in Formula 1;
$R_3$ is

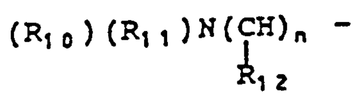

wherein $R_{10}$, $R_{11}$ are hydrogen or alkyl preferably $C_1$-$C_3$ alkyl, $R_{12}$ is hydrogen, alkyl preferably $C_1$-$C_3$alkyl, or aryl, with or without heteroatoms,
or $R_{11}$, $R_{12}$, form a ring, and
n is an integer from 1 to 5;

2c $R_1$, $R_2$, and Ar are as defined in Formula 1;
$R_3$ is hydrogen, or as in 2a, or as in 2b; and
$R_5$ is $CO_2R_8$, wherein $R_8$ is hydrogen or alkyl.

Preferred are compounds wherein Ar is 2- or 3-thienyl, 1-napthyl or 2-napthyl, 3-thianapthenyl or especially phenyl optionally mono- or poly-substituted by halogen, alkyl, alkoxy or trifluoromethyl.

Compounds of the invention are inhibitors of the enzyme 5-lipoxygenase and, accordingly, may be employed in the treatment of inflammation and in diseases where products of 5-lipoxygenase metabolism play a role, such as asthma, psoriasis, rheumatoid arthritis and inflammatory bowel disease. Compounds of formula 1' also inhibit the enzyme cyclooxygenase, although they are generally more potent in inhibiting 5-lipoxygenase.

The prior art contains references for six compounds of general Formula 1 wherein Ar is a substituted phenyl ring or pyridyl ring and $R_1$ and $R_2$ are both $C_1$-$C_3$ alkyl, and one compound wherein $R_1$ is allyl $R_2$ is methoxy and Ar is phenyl. For example, one prior art compound ($R_1$ and $R_2$ are methyl, and Ar is 4-hydroxy-3-methoxyphenyl) was disclosed as generated by the treatment of spruce wood meal with alkali (2.2M NaOH) or white liquor (2.1 M NaOH, 0.2 M $Na_2S$) in the presence of 2,6-xylenol (J. Gierer et al., Acta.

Chem. Scand., Ser. B, 1979, B33 (8), 580. C.A. 92:78366t, 1980). Another prior art compound ($R_1$ and $R_2$ are methyl, and Ar is 3,5-dimethyl-4-hydroxyphenyl) is one of many symmetrical stilbene derivatives synthesized by condensation of chloroacetaldehyde with aromatic compounds, followed by re-arrangement. (R.H. Sieber, Justus Liebigs Ann. Chem. 1969, 730, 31). The oxidation of the 2,6-dialkyl-4-methylphenol with silver carbonate, followed by reduction of the resulting stilbene quinone with zinc in acetic acid is also reported as generating such prior art compound and a further prior art compound wherein $R_1$ and $R_2$ are isopropyl and Ar is 3,5-diisopropyl-4-hydroxyphenyl (V. Balogh et al. J. Org. Chem. 1971, 36, 1339).

Compounds of formula I wherein Ar is p-pyridyl, G is hydroxy, $R_1$ is methyl, iso-propyl or tert. butyl and $R_2$ is iso-propyl, tert. butyl or cyclohexyl and $R_5$ is hydrogen or methyl, and compounds of formula I wherein Ar is o-pyridyl, G is hydroxy, $R_1$ is methyl, iso-propyl or tert. butyl, $R_2$ is iso-propyl or tert. butyl and $R_5$ is hydrogen are described in Khim. Geterotsikl. Soedin. 1971, 7(12), 1660-4 reported in CHEMICAL ABSTRACTS, Vol. 76, No. 25, June 19, 1972, Columbus, Ohio, USA, page 445, column 1, abstract no. 153 516s. A compound of formula I, wherein G is hydroxy, $R_1$ and $R_2$ are methyl and Ar is 3-methoxy-4-hydroxyphenyl is described in Acta Chem. Scand., Ser. B 1979, B 33(8), 580-2 reported in CHEMICAL ABSTRACTS, Vol. 92, No. 9, March 3, 1980, Columbus, Ohio, USA, page 105, column 2, abstract no. 78 366t.

The synthesis of prior art compounds wherein $R_1$ and $R_2$ are methyl, or $R_1$ is allyl and $R_2$ is methoxy, and Ar is phenyl by Grignard reaction of the phenolic aldehyde with benzyl magnesium bromide followed by dehydration with potassium hydrogen sulfate has been reported (H.D. Becker, J. Org. Chem. 1969, 34, 1211). The compounds are used as intermediates for dehydrogenation to bisquinone methides.

Two prior art pyridyl compounds ($R_1$ and $R_2$ are isopropyl, and Ar is 2-pyridyl or 4-pyridyl) are reportedly synthesized by condensation of the phenolic aldehyde with N-acyl picolinium salts (G.N. Bogdanov et al., Khim Geterotsikl. Soedin., 1971, 7, 1660 CA 76: 153516, 1972). Such compounds are reported to be useful as antioxidants and useful as anti-tumor agents.

Certain derivatives of 2,6-di-t-butylphenol are reported to be dual inhibitors of the enzymes cyclooxygenase and 5-lipoxygenase. All of these are more potent inhibitors of cyclooxygenase than lipoxygenase, and accordingly useful primarily as cyclooxygenase-inhibiting, non-steroidal anti-inflammatory agents. These 2,6-di-t-butylphenol derivatives include compounds having the following formulas shown below: 3 (G.G.I. Moore and K.F. Swingle, Agents Actions, 1982, 12, 674.); 4 (T. Hidaka et al., Jap. J. Pharmacol. 1984, 36, 77.); 5 (H. Shirota et al., Third International Conference, Inflammation Research Association Poster Session, Nos. 15 and 16, White Haven, Pa. 1986); and 6 (E. Lazer, U.S.S.N. 843,898).

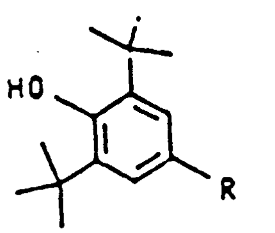

3 R = 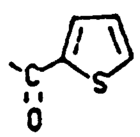

5 R = 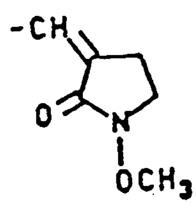

4 R = 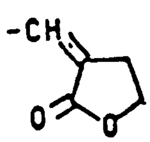

6 R = 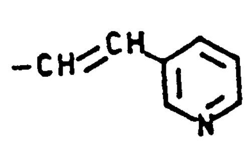

The compounds of the present invention distinguish themselves from compounds having formulas 3 through 6 by being more potent and selective inhibitors of 5-lipoxygenase.

According to another aspect of the present invention there is provided a process for the preparation of compounds of the present invention which comprises

a) to prepare a compound of formula 1 wherein $R_5$ is hydrogen and G is -OH, reacting a compound of formula 7

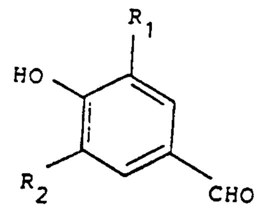

7

with an aryl or heteroaryl acetic acid 8

$ArCH_2CO_2H$ 8

to yield a compound of formula 1" having the structure

1"

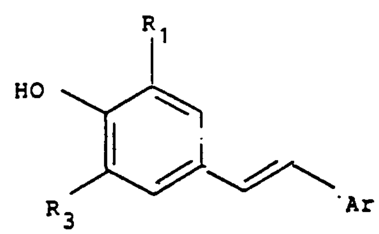

wherein Ar, $R_1$ and $R_2$ are as defined above,
b) to prepare a compound of formula 1, wherein $R_5$ is $CO_2R_8$ and G is -OH, reacting a compound of formula 7 as defined above with an aryl or heteroaryl acetic acid ester having the formula 9

$ArCH_2CO_2R_8$ 9

to yield a compound having formula 10

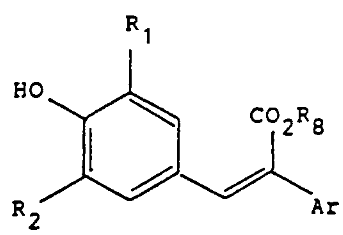

10

wherein Ar, $R_1$, $R_2$ are as defined above and $R_8$ is $C_1$-$C_3$ alkyl,
c) to provide a compound of formula 1 wherein $R_8$ is hydrogen, hydrolysing an ester of formula 10'

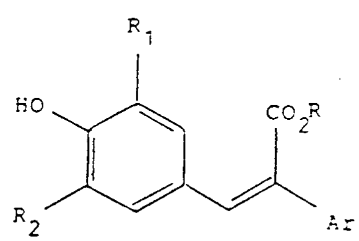

10'

wherein R, which may be $R_8$ when this is $C_1$-$C_3$ alkyl, is an ester forming group capable of being removed in a manner known per se,
to yield the free acid 11

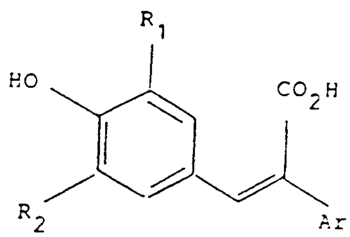

11

wherein Ar, $R_1$ and $R_2$ are as defined above:
d) to provide a compound of formula 1 wherein $R_5$ is hydrogen, decarboxylation of an acid of formula 11 to yield a compound of formula 1"
e) to yield a compound of formula 1 wherein $R_5$ is $C_1$-$C_5$ alkyl, deprotecting a compound having the formula 12

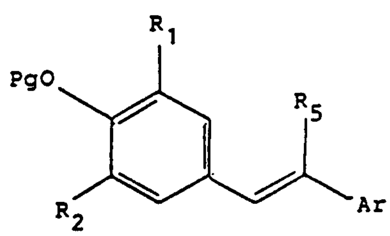

12

wherein $R_1$, $R_2$ and Ar are as defined above and Pg is a hydroxy protecting group
f) to yield a compound of formula 13

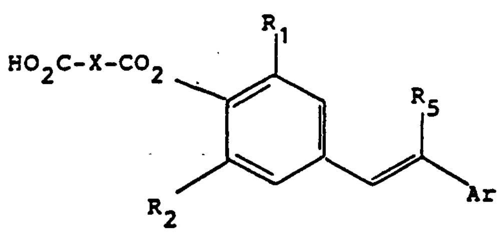

13

reacting a compound of the formula wherein G is OH with a compound of the formula

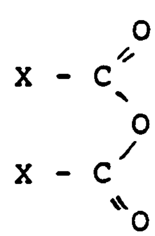

g) to prepare a compound of formula 1 wherein G is the group

$$(R_{10})(R_{11})N(\underset{R_{12}}{CH})_n-CO_2- \qquad \mathbf{15}$$

as defined above,
reacting a compound of the formula 1 wherein G is OH and $R_5$ is alkyl, or a compound of formula 10' as defined above with a compound of the formula

$$(R_{10'})(R_{11'})N(\underset{R_{12}}{CH})_nCOOH \qquad \mathbf{16}$$

wherein $R_1$, $R_2$ and $R_5$ are as defined above, $R_{10'}$ and $R_{11'}$ are hydrogen or alkyl with the proviso that if one is hydrogen the other is alkyl or a removable protecting group, and $R_{12}$ is hydrogen, alkyl or aryl which may contain at least one heteroatom, and wherein $R_{11'}$ and $R_{12}$ may together form a ring, and n is an integer from one to five, and thereafter removing the groups $R_{10'}$ and/or $R_{11'}$ when these are protecting groups and if necessary or desired removing the group R;
and thereafter, if desired, treating the product of process a) to g) by one or more of the following finishing steps in any desired order:
i) removing an ester group $R_8$ to yield a free acid
ii) esterifying a free acid to yield a pharmaceutically acceptable ester wherein the ester group is capable of being cleaved in vivo to yield the free acid
iii) forming a pharmaceutically acceptable salt of the free acid e.g. the salt of an alkali metal or alkaline earth metal e.g. sodium, potassium or calcium
iv) forming an acid addition salt of a compound of formula 1 in particular such a compound wherein $R_3$ is the group

$$(R_{10})(R_{11})N(\underset{R_{12}}{CH})_n -$$

Thus the compounds of the present invention can be prepared by reaction of an appropriately substituted phenolic aldehyde 7 with an aryl- or heteroaryl acetic acid 8 in the presence of a basic catalyst, as shown below. A suitable catalyst would be for example piperidine, morpholine or triethylamine. The reaction my be performed in the presence of an inert solvent, such as methylene chloride, chloroform or benzene, which may be added at the beginning of the reaction to facilitate mixing, and then boiled off as the reaction progresses. The reaction may also be carried out in a refluxing solvent, such as toluene, xylene, or ethylenedichloride.

The compounds of the invention may also be prepared by reaction of 7 with an aryl- or heteroaryl acetic acid ester 9 in the presence of a basic catalyst and an inert solvent, as shown below. Again, a suitable catalyst would be piperidine. An inert solvent could be ethanol. The resulting ester 10 is hydrolyzed to carboxylic acid 11, which is decarboxylated to yield 1. Intermediates 10 and 11 can also be prodrugs of formula 2c, the prodrug described above.

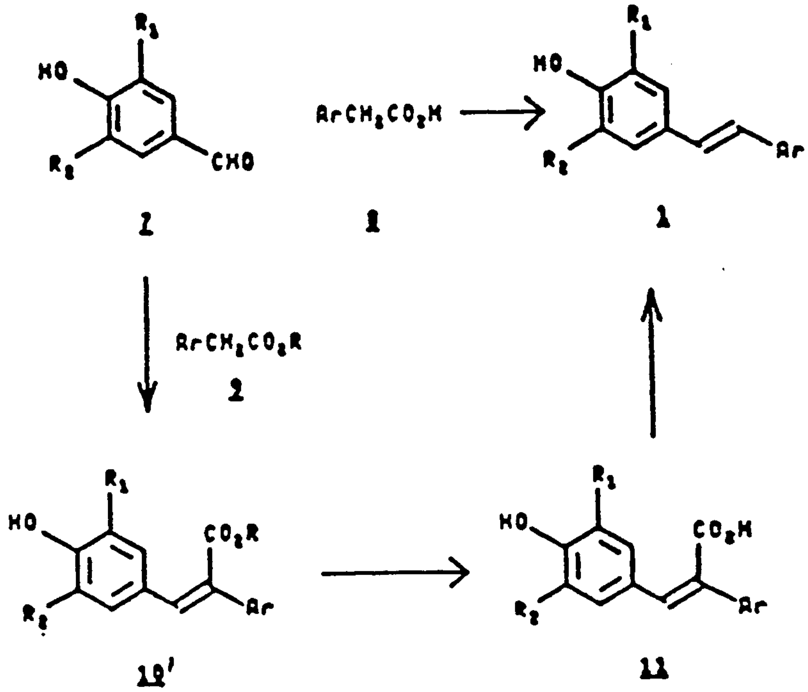


7 8 1 9 10' 11

The required substituted phenolic aldehydes (7) are prepared from the corresponding substituted phenols (12) by reaction of the phenols with hexamethylenetetramine in the presence of an acid catalyst. [Duff reaction, J.C. Duff, J. Chem Soc. 574 (1941)]. As examples, the acid used may be acetic acid, which can be used as the reaction solvent, or boric acid in ethylene glycol as solvent followed by hydrolysis. These modified conditions for the Duff reaction are reported in the literature. The required phenols (12) and arylacetic acids (7) are available commercially or may be prepared by methods reported in the literature.

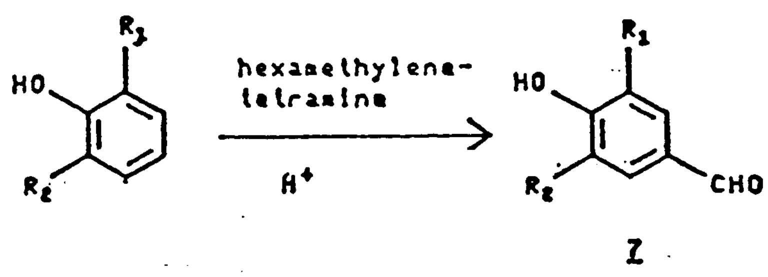


7

For the compounds of formula I where $R_5$ is methyl or n-butyl, the compounds may be prepared via a Wittig reaction; i.e., a protected phenolic aldehyde (e.g., an ethyl carbonate derivative) is reacted with a Wittig salt of the corresponding phenyl derivative with a base catalyst, such as n-butyllithium, in a suitable solvent such as ether, followed by deprotection e.g. by hydrolysis of the carbonate, to yield the phenol. The remaining compounds of formula I may be prepared using materials and techniques well within the ability of one skilled in the art.

The prodrug esters of formula 2a may be prepared by standard procedures, for example by reaction of a phenol of formula 1 with an anhydride in the presence of a base in an inert solvent. The prodrug esters of formula 2b may be prepared by standard procedures, for example by reaction of a phenol of formula 1 with a protected amino acid in the presence of a coupling reagent such as dicyclohexylcarbodiimide in an inert solvent. A basic catalyst such as 4-dimethylaminopyridine may be added. The protecting group is then removed from the amino acid by standard procedures. The protecting group used may be one of those generally employed in peptide chemistry. For example, tert-butyloxycarbonyl, benzyloxycarbonyl, 9-fluorenylmethyloxycarbonyl or biphenylisopropyloxycarbonyl may be used as amino-protecting groups. See The Practice of Peptide Synthesis, M. Bodanszky and A. Bodanszky (Springer-Verlag, 1984). The prodrugs

of formula 2c may be formed by reaction of an aldehyde of formula 7 with an arylacetic ester, formula 9, in a refluxing solvent in the presence of a basic catalyst. A suitable refluxing solvent is ethanol, for example. A suitable basic catalyst is piperidine, for example.

The compounds according to the present invention may be administered to warm-blooded animals topically, perorally, parenterally, rectally or by the respiratory route as active ingredients in conventional pharmaceutical compositions, that is, compositions comprising a pharmaceutical carrier or excipient and an effective amount of the active ingredient.

In another aspect, the present invention provides pharmaceutical compositions comprising a compound of formula I or a physiologically acceptable salt thereof, together with at least one pharmaceutical carrier or excipient.

In a yet still further aspect the invention provides the use of a compound of formula I or a physiologically acceptable salt thereof for the preparation of a pharmaceutical composition for the treatment of allergic or inflammatory reactions of humans or other warm-blooded animals.

When the compounds of the invention are given by the oral route, they may be formulated in the form of syrups, tablets, capsules, pills and the like. Preferably, the compositions are in unit dosage form, or in a form in which the patient can administer to himself a single dose. When the composition is in the form of a tablet, powder or lozenge, any pharmaceutical carrier suitable for formulating solid compositions may be used. Examples of such carriers are various starches, lactose, glucose, sucrose, cellulose, dicalcium phosphate, and chalk. The composition may also be in the form of an ingestible capsule (for example of gelatin) containing the compound; or in the form of a syrup, a liquid solution or a suspension. Suitable liquid pharmaceutical carriers include ethyl alcohol, glycerin, saline, water, propylene glycol or sorbitol solution, which may be compounded with flavoring or coloring agents to form syrups.

The compounds of this invention may also be administered by other than the oral route. In accordance with routine pharmaceutical procedure, the compositions may be formulated, for example, for rectal administration as a suppository or for presentation in an injectable form in an aqueous or non-aqueous solution, suspension or emulsion in a pharmaceutically acceptable liquid, such as sterile, pyrogen-free water of a parenterally acceptable oil or a mixture of liquids, which may contain bacteriostatic agents, antioxidants, preservatives, buffers, or other solutes to render the solution isotonic with the blood, thickening agents, suspending agents or other pharmaceutically acceptable additives. Such forms will be presented in unit dose forms such as ampules or disposable injection devices or in multi-dose vials such as a bottle from which the appropriate dose may be withdrawn, or in solid form or concentrate which can be used to prepare an injectable formulation.

Compounds of this invention may also be suitably presented for administration to the respiratory tract as an aerosol or solution for a nebulizer, or as a microfine powder for insufflation, alone or in combination with an inert carrier such as lactose. In such a case the particles of active compounds suitably have diameters of less than 20 microns, preferably less than 10 microns. Where appropriate, small amounts of other anti-allergics, anti-asthmatics and bronchodilators, for example, sympathomimetic amines such as isoprenaline, isoetharine, metaproterenol, salbutamol, phenylephrine, fenoterol and ephedrine; xanthine derivatives such as theophylline and aminophylline; corticosteroids such as prednisolone and adrenal stimulants such as ACTH may be included.

Compounds of this invention may also be presented as an ointment, cream, lotion, gel, aerosol or solution for topical application to the skin, nose, or eye.

For the preparation of pharmaceutical compositions, the compounds of the invention are mixed in the usual way with appropriate pharmaceutical carrier substances and aroma, flavoring and coloring materials and formed, for example, into tablets or capsules or, with the addition of appropriate adjuvants, suspended or dissolved in water or in an oil, for example corn oil.

The compounds of this invention can be administered orally and parenterally in liquid or solid form. As injection medium, it is preferred to use water which contains the stabilizing agents, solubilizing agents and/or buffers conventionally used for injection solutions. Additives of this type include, for example, tartrate, citrate and acetate buffers, ethanol, propylene glycol, polyethylene glycol, complex formers (such as EDTA), antioxidants (such as sodium bisulfate, sodium metabisulfate or ascorbic acid), high-molecular-weight polymers (such as liquid-polyethylene oxides) for viscosity regulation, and polyethylene derivatives of sorbitol anhydrides.

Preservatives may also be added, if necessary, such as benzoic acid, methylene propylparaben, benzalkonium chloride or other quaternary ammonium compounds.

Solid carrier materials which can be used include, for example, starch, lactose,mannitol, methyl cellulose, microcrystalline cellulose, talcum, pyrogenic silica, dicalcium phosphate, and high-molecular-weight polymers (such as polyethylene glycol).

The following examples demonstrate reaction conditions under which compounds of the present invention may be prepared. Such examples are illustrative only, and do not limit the scope of the present invention.

Example 1

3,5-Dimethyl-4-hydroxybenzaldehyde

2,6-Dimethylphenol (50g, 0.41 mol) and hexamethylenetetramine (57g, 0.41 mol) were combined in 400 mL acetic acid and heated to reflux for 4.5 hours. The reaction mixture was poured into 1500 mL water and stirred until a precipitate formed. The mixture was refrigerated overnight, filtered, and the solid product dried and recrystallized from EtOH. Two crops of 3,5-Dimethyl-4-hydroxybenzaldehyde were obtained for a total of 19.02g (0.127 mol, 31%), mp 114-115 °C

Example 2

The compound in Example 1 may also be prepared under the following conditions: 2,6-Dimethylphenol (100g, 0.818 mol), hexamethylenetetramine (195g, 1.39 mol) and boric acid (270g, 4.37 mol) were combined in 1,000 mL ethylene glycol and heated at 130 °C, with stirring, for 1 hr. The hot reaction mixture was combined with 1,400 mL 30% $H_2SO_4$ and stirred overnight at ambient temperature. The precipitated product was filtered, rinsed with water and recrystallized from EtOH giving 85g, 3,5-dimethyl-4-hydroxybenzaldehyde (0.54 mol, 66%), mp 112-113 °C

Example 3

3-Ethyl-5-methoxy-4-hydroxybenzaldehyde

2-Ethyl-6-methoxyphenol (11.4g, 0.075 mol) and hexamethylenetetramine (10.5g. 0.075 mol) were combined in 65 mL acetic acid and heated to reflux for 4.5. hours. The reaction mixture was poured onto ice and water, and the product extracted into $CH_2Cl_2$ (3 x 150 mL). The combined organic extracts were washed with saturated NaCl solution, dried ($Na_2SO_4$) and concentrated in vacuo. The product was recrystallized from EtOH giving 2.53g. Crude product from the mother liquor was purified by column chromatography on silica gel, eluting with $CH_2Cl_2$, giving another 2.1 g for a total of 4.63 g, (0.026 mol, 34%).

The following examples are illustrative of the general procedures used to prepare the compounds of this invention (General Formula 1) which are listed in Table 1.

Example 4

2,6-Dimethyl-4-[2-(2-thienyl)ethenyl]phenol

Piperidine (58g, 0.68 mol) was added to 2-thiopheneacetic acid (34g, 0.24 mol) suspended in 75 mL $CH_2Cl_2$. 3,5-Dimethyl-4-hydroxybenzaldehyde (30g, 0.2 mol) was added and the mixture was heated in an oil bath (bath temperature: 130 °C) with mechanical stirring, under $N_2$. The $CH_2Cl_2$ was allowed to boil off. After heating for 7 hours, the dark brown residue was eluted through a silica gel column with $CH_2Cl_2$. The major fraction was concentrated, and the product recrystallized from toluene giving 21.5g 2,6-Dimethyl-4-[2-(2-thienyl)ethenyl]phenol, (0.093 mol, 47%) mp 133-134 °C.

| Anal: Calcd. for $C_{14}H_{14}OS$: | | | |
|---|---|---|---|
| | C 73.01; | H 6.13; | S 13.92 |
| Found: | C 73.04; | H 6.02; | S 13.98 |

'H NMR: 2.3 (6H, s, $CH_3$), 4.7 (1H, s, OH), 6.75-7.2 (7H, aromatic and olefinic protons).

Example 5

The product of Example 4 may also be prepared by the following procedure:

2-Thiopheneacetic acid (15g, 0.105 mol) and piperidine (12.9g, 0.15 mol) were combined in 450 mL to and stirred for 20 minutes. 3,5-Dimethyl-4-hydroxybenzaldehyde (15g 0.100 mol) was added and the reaction mixture heated to reflux with a Dean-Stark trap attached to remove water formed in the reaction. After 20 hours, the mixture was cooled to room temperature and poured onto a pad of silica gel packed 3/4 full onto a 2L fritted glass filtering funnel. The product was eluted with toluene and the light yellow solid was recrystallized from ligroine giving 14g 2,6-dimethyl-4-[2-(2-thienyl)ethenyl]phenol,(0.061 mol, 61%), mp 133-134 ° C.

| Anal: Calcd. for $C_{14}H_{14}OS$: | | | |
|---|---|---|---|
| | C 73.01; | H 6.13; | S 13.92 |
| Found: | C 73.19; | H 6.18; | S 13.97 |

'H NMR: 2.3 (6H, s, $CH_3$), 4.7 (1H, s, OH), 6.75-7.2 (7H, aromatic and olefinic protons).

Example 5a

Ethyl 3-(3,5-dimethyl-4-hydroxyphenyl)-2-(2-thienyl)propenoate

A mixture of 3,5-dimethyl-4-hydroxybenzaldehyde (15g, 0.1 mol), ethyl 2-thiopheneacetate (17.9 g, 0.105 mol), piperdine (9g, 0.105 mol) and p-toluenesulfonic acid (100 mg) in 300 mL EtOH was heated to reflux for 18 hours. The reaction mixture was concentrated down to about half its original volume on a rotary evaporator, and a precipitate formed. The mixture was stored in the freezer overnight, filtered and the solid rinsed with cold EtOH. The solid was then suspended in water, the mixture made acidic with 1N HCl and the solid filtered and dried giving 12.2g ethyl 3-(3,5-dimethyl-4-hydroxyphenyl)-2-(2-thienyl) propenoate, (0.04 mol, 40%) mp 85-87 ° C. A small sample was recrystallized from EtOH, mp 87-89 ° C.

| Anal: Calcd. for $C_{17}H_{18}O_3S$: | | | |
|---|---|---|---|
| | C 67.52 | H 6.00 | S 10.60 |
| Found: | C 67.64 | H 6.00 | S 10.62 |

'H NMR; 1.3 (3H, t, $CH_3$), 2.05 (6H, s, $CH_3$), 4.26 (2H, q. $CH_2$), 5.0 (1H, s, OH), 6.8-7.8 (6H, aromatic and olefinic protons).

Example 5b

3-(3,5-Dimethyl-4-hydroxyphenyl)-2-(2-thienyl)propenoic acid

A mixture of ethyl 3-(3,5-dimethyl-4-hydroxyphenyl)-2-(2-thienyl)-propenoate (1.5g, 5 mmol) 10 mL 2N NaOH and 10 mL EtOH was heated to reflux for 3.5 hours. The reaction mixture was then concentrated to half its original volume and combined with 15 mL 2N HCl while stirring on an ice bath. The resulting solid was filtered, dried and recrystallized from toluene giving 0.97g 3-(3,5-dimethyl-4-hydroxyphenyl)-2-(2-thienyl) propenoic acid (3.5 mmol, 71%), mp 195-197 ° C.

| Anal: Calcd. for $C_{15}H_{14}O_3S$: | | | |
|---|---|---|---|
| | C 65.67 | H 5.14 | S 11.69 |
| Found: | C 65.60 | H 5.08 | S 11.60 |

'H NMR: 2.0 (6H, s, $CH_3$), 6.7-7.7 (6H, aromatic and olefinic protons), 8.8 (1H, s, OH), 12.6 (1H, s, $CO_2H$).

Example 5c

The product of Example 4 may also be prepared by the following procedure:

3-(3,5-dimethyl-4-hydroxyphenyl)-2-(2-thienyl)propenoic acid (5.3g, 19.3 mmol) was combined with piperidine (1.7g, 20 mmol), forming a light brown mass. Toluene (200 mL) was added and the reaction stirred at room temperature for 20 minutes and then refluxed for 40 hours. The reaction mixture was concentrated to half its original volume and passed through a glass funnel containing silica gel (250g) and eluted with toluene. The light yellow fractions containing product were concentrated giving 1.98g 2,6-dimethyl-4-[2-(2-thienyl)ethenyl]phenol (1a, Table 1), (8.6 mmol, 44% mp 132-133°C). The 'H NMR spectrum was identical to the one in Example 4.

Example 6

2-Methoxy-6-methyl-4-[2-(2-thienyl)ethenyl]phenol

Piperidine (2.7g, 32 mmol) was added to a suspension of 2-thiopheneacetic acid (3.3g, 23 mmol) in 10 mL $CH_2Cl_2$, followed by 3.5g (21 mmol) 4-hydroxy-3-methoxy-5-methylbenzaldehyde. The stirred mixture was heated on an oil bath, the temperature of the oil bath was allowed to rise to 135°C over 3-4 hr. After 2 hours additional piperidine (2.6g, 30 mmol) was added. The reaction mixture was removed from the oil bath after about 4.5 hours. The residue was taken up in 100 mL EtOAc, and washed with 1N HCL (100 mL), water (100 mL), saturated $NaHCO_3$ (100 mL), 1N HCl (75 mL), saturated NaCl solution (100 mL), dried ($Na_2SO_4$) and concentrated. The residue was recrystallized twice from EtOH giving 0.95g 2-methoxy-6-methyl-4-[2-(2-thienyl)ethenyl]phenol (3.9 mmol, 18%), mp 113-114°C.

| Anal: Calcd. for $C_{14}H_{14}O_2S$: | | | |
|---|---|---|---|
| | C 68.26 | H 5.74 | S 13.02 |
| Found: | C 68.63 | H 5.78 | S 12.89 |

'H NMR: 2.25 (3H, s, $CH_3$), 3.9 (3H, s, $CH_3$), 5.7 (1H, s, OH), 6.8-7.2 (7H, aromatic and olefinic protons).

Example 7

2,6-Diethyl-4-[2-(2-thienyl)ethenyl]phenol

A mixture of 3,5-diethyl-4-hydroxybenzaldehyde (2.5g, 14 mmol), 2-thiopheneacetic acid (2.58g, 18.2 mmol) and piperidine (4.3g, 51 mmol) in 20 mL $CH_2Cl_2$ was heated in an oil bath (130-140°C) for 2.5 hours. The reaction mixture was taken up in EtOAc (100 mL), washed with water (100 mL), the saturated $NaHCO_3$ solution (2 x 50 mL), saturated NaCl (50 mL), dried ($Na_2SO_4$) and then concentrated. The residue was passed through a silica gel column, eluting with $CH_2Cl_2$, the fractions containing product concentrated and the residue recrystallized from EtOH giving 1.57g 2,6-Diethyl-4-[2-(2-thienyl)ethenyl]phenol (6.1 mmol, 43%), mp 113-115°C.

| Anal: Calcd. for $C_{16}$ $H_{18}$ OS:; | | | |
|---|---|---|---|
| | C 74.38 | H 7.02 | S 12.41 |
| Found | C 74.24 | H 7.03 | S 12.36 |

'H NMR: 1.25 (6H t, $CH_3$), 2.6 (4H, q, $CH_2$), 4.75 (1H, s, OH), 6.8-7.2 (7H, aromatic and olefinic protons).

The following two examples illustrate procedures by which a prodrug of type 2a or 2b may be prepared.

Example 8

2,6-Dimethyl-4-[2-(2-thienyl)ethenyl]phenyl succinate

1.47g 4-Dimethylaminopyridine (12 mmol), 1g succinic anhydride (10 mmol), and 1g triethylamine (10 mmol) were added to a solution of 1a (Table 1) (2.3g, 10 mmol) in 25 mL $CH_2Cl_2$. After stirring at room

temperature for 2.5 hours the reaction was heated to reflux for 1 hour. Then additional 4-dimethylaminopyridine (0.5g), succinic anhydride (0.3g) and triethylamine (1g) were added and the reaction stirred at room temperature another 30 minutes. The reaction was diluted with 50 mL $CH_2Cl_2$, washed with 1N HCl (2 x 50 mL), saturated NaCl (50 mL), dried ($Na_2SO_4$) and concentrated. The residue was recrystallized from isopropanol giving 2.25g 2.6-Dimethyl-4-[2-(2-thienyl)ethenyl]phenyl succinate (6.8 mmol, 68%) mp 168-170 ° C

| Anal: Calcd. for $C_{18}H_{18}O_4S$: | | | |
|---|---|---|---|
| | C 65.43 | H 5.50 | S 9.70 |
| Found: | C 65.28 | H 5.48 | S 9.60 |

'H NMR: 2.2(6H, s, $CH_3$), 2.9 (4H, m, $CH_2CH_2$), 6.75-7.3 (7H, aromatic and olefinic protons), 11 (1H, broad s, $CO_2H$).

Example 9

2,6-Dimethyl-4-[2-(2-thienyl)ethenyl]phenyl glycinate hydrochloride

A mixture of 1a (Table 1) (4.6g, 20 mmol), N-(tert-butoxycarbonyl)glycine (3.5g, 20 mmol), 4-dimethylaminopyridine (0.8g, 6.5 mmol) and dicyclohexylcarbodimide (4.2g, 20 mmol) in 175 mL $CH_2Cl_2$ was stirred for 16 hours at room temperature. The white precipitate was filtered off and rinsed with $CH_2Cl_2$. The filtrate was washed with 1N HCl (2 x 75 mL), saturated $NaHCO_3$ solution (1 x 75 mL) and saturated NaCl solution (1 x 75mL) dried ($Na_2SO_4$) and concentrated. The residue was recrystallized from ethanol giving 5.6g of the protected glycine ester (14.5 mmol, 72% mp 124-125 ° C).

3.8g of the protected glycine ester (9.8 mmol) was dissolved in 250 mL anhydrous ether and HCl gas was bubbled in, with stirring for 30 minutes. The reaction was stirred for 18 hours at room temperature, then the precipitated product was filtered. After stirring the filtrate another 24 hours, additional product was collected for a total of 3.1g 2,6-dimethyl-4-[2-(2-thienyl)ethenyl]phenyl glycinate hydrochloride (9.6 mmol, 98%) mp 275-277 ° C.

| Anal: Calcd. for $C_{16}H_{17}NO_2S \cdot HCl$: | | | | | |
|---|---|---|---|---|---|
| | C 59.34 | H 5.60 | Cl 10.95 | N 4.33 | S 9.90 |
| Found: | C 58.97 | H 5.64 | Cl 11.38 | N 4.38 | S 9.72 |

'H NMR: 2.1 (6H, s, $CH_3$), 4.25 (2H, s, $CH_2$), 6.8-7.5 (7H, aromatic and olefinic protons), 8.6 (3H, s, $NH_3^+$).

Inhibition of 5-Lipoxygenase in Human Polymorphonuclear Leukocytes (PMN's)

The inhibition of 5-lipoxygenase is measured by determining whether and to what extent test compounds inhibit the amount of 5-HETE biosynthesized by human PMN's. 5-HETE biosynthesis serves as a marker for arachidonic acid metabolism by 5-lipoxygenase.

Human PMN's (5 x $10^6$ cells/0.5 mL) in pH 7.2 phosphate buffer containing $Ca^{2+}$ (0.6 mM) and $Mg^{2+}$ - (1.0 mM) were incubated with test compound for 15 minutes at 37 ° C with shaking. Calcium ionophore A23187 (0.25 mM, 0.01 mL) and [$^{14}C$] arachidonic acid (0.10 microCi in 0.025 mL 0.01N NaOH) were added, and the mixture incubated another 2.5 minutes. The reaction was terminated by the addition of 0.025 mL of 1.0 N HCl. The mixture was then extracted with ethyl acetate-methylene chloride (2:3) supplemented with 12 microgram/mL cold arachidonic acid to reduce degradation of the metabolites. After concentrating the organic phase, the number of microlitres containing 5 x $10^4$ cpm was determined and that volume applied to a silica gel plate. The plate was developed in methylene-chloride-methanol-acetic acid-water (90:8:1:0.8), air dried, and counted on a Berthold linear TLC analyzer. The integrated area of the 5-HETE band was determined and compared with the control (no drug).

Table 1 shows the percent inhibition of 5-lipoxygenase by compounds of the invention at test concentrations of 1 microM. In cases where the $IC_{50}$ was determined, the result is shown in microM.

Table 1: Inhibition of 5-lipoxygenase by Compounds of General Formula 1

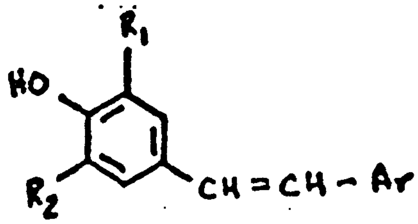

**TABLE 1**

| Compound No. | $R_1$ | $R_2$ | X | Formula | mp(C°) | Inhibition of 5-LO % at 1 M | $IC_{50}$ ( M) |
|---|---|---|---|---|---|---|---|
| Ar= 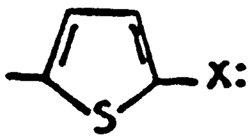 | | | | | | | |
| 1a | $CH_3$ | $CH_3$ | H | $C_{14}H_{14}OS$ | 133-134 | 91 | 0.07 |
| 1b | $CH_3$ | $CH_3$ | Cl | $C_{14}H_{13}ClOS$ | 125-126 | 77 | |
| 1c | $CH_3$ | $CH_3$ | $CH_3$ | $C_{15}H_{16}OS$ | 104-105 | 62 | |
| 1d | $CH_3$ | $OCH_3$ | H | $C_{14}H_{14}O_2S$ | 113-114 | 65 | 0.08 |
| 1e | $CH_3CH_2$ | $OCH_3$ | H | $C_{15}H_{16}O_2S$ | 85.5-86.5 | 85 | 0.11 |
| 1f | $CH_3CH_2CH_2$ | $OCH_3$ | H | $C_{16}H_{18}O_2S$ | 99-100 | 76 | 0.14 |
| 1g | $CH_3$ | $OCH_3$ | Cl | $C_{14}H_{13}ClO_2S$ | 90-91 | 76 | |
| 1h | $CH_2{=}CH{-}CH_2$ | $CH_3$ | H | $C_{16}H_{16}OS$ | 79-80 | 70 | 0.1 |
| 1i | $CH_3CH_2$ | $CH_3CH_2$ | H | $C_{16}H_{18}OS$ | 113-115 | 70 | |
| 1j | $(CH_3)_2CH$ | $(CH_3)_2CH$ | H | $C_{18}H_{22}OS$ | 45.5-48.5 | 78 | 0.6 |
| 1k | $CH_3$ | F | H | $C_{13}H_{11}FOS$ | 86-88 | 65 | |
| 1l | $CH_3$ | Cl | H | $C_{13}H_{11}ClOS$ | 109.5-110.5 | 84 | |
| 1m | $OCH_3$ | F | H | $C_{13}H_{11}FO_2S$ | 82-83 | 65 | |
| 1n | $OCH_3$ | $OCH_3$ | H | $C_{14}H_{14}O_3S$ | 95-96.5 | 78 | |

| Compound No. | $R_1$ | $R_2$ | Formula | mp(C°) | Inhibition of 5-LO % at 1 M | $IC_{50}$ ( M) | p |
|---|---|---|---|---|---|---|---|
| Ar= 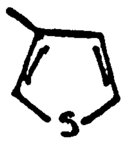 | | | | | | | |
| 1o | $CH_3$ | $CH_3$ | $C_{14}H_{14}OS$ | 144-145 | 84 | 0.07 | |
| 1p | $CH_3$ | $OCH_3$ | $C_{14}H_{14}O_2S$ | 128-130 | 70 | | |
| 1q | $OCH_3$ | $OCH_3$ | $C_{14}H_{14}O_3S$ | 97-98 | 77 | | |
| 1r | $CH_3CH_2$ | $OCH_3$ | $C_{15}H_{16}O_2S$ | 122-123.5 | 82 | | |
| 1s | $CH_3$ | Cl | $C_{13}H_{11}ClOS$ | 104-105 | 75 | | |
| 1t | $OCH_3$ | F | $C_{13}H_{11}FO_2S$ | 101-103 | 59 | | |
| 1u | $CH_3CH_2$ | $CH_3CH_2$ | $C_{16}H_{18}OS$ | 130-132 | 66 | | |
| 1v | $CH_2=CHCH_2$ | $CH_3$ | $C_{16}H_{16}OS$ | 97-99 | 75 | | |
| Ar = 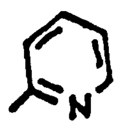 (p=2), 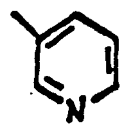 (p=3), or 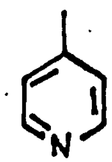 (p=4): | | | | | | | |
| 1w | $CH_3$ | $CH_3$ | $C_{15}H_{15}NO$ | 163-164 | 77 | | p=2 |
| 1x | $CH_3$ | $CH_3$ | $C_{15}H_{15}NO$ | 144-145 | 62 | 0.5 | p=3 |
| 1y | $CH_3$ | $CH_3$ | $C_{15}H_{15}NO$ | 208-209 | 46 | | p=4 |
| 1z | $CH_3$ | $OCH_3$ | $C_{15}H_{15}NO_2$ | 144-145 | 52 | 0.9 | p=3 |
| 1aa | $CH_3CH_2$ | $CH_3CH_2$ | $C_{17}H_{19}NO$ | 130-131 | 59 | | p=3 |
| 1ab | $(CH_3)_2CH$ | $(CH_3)_2CH$ | $C_{19}H_{23}NO$ | 136-138 | 73 | 0.6 | p=3 |
| 1ac | $CH_2=CHCH_2$ | $CH_3$ | $C_{17}H_{17}NO$ | 111-113 | 61 | | p=3 |

| Compound No. | $R_1$ | $R_2$ | X | Formula | mp(C°) | Inhibition of 5-LO % at 1 M | $IC_{50}$ ( M) |
|---|---|---|---|---|---|---|---|
| Ar= 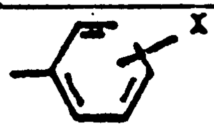 | | | | | | | |
| lad | $CH_3$ | $CH_3$ | H | $C_{16}H_{16}O$ | 139-141 | 72 | 0.18 |
| lae | " | " | 4-$CH_3$ | $C_{17}H_{18}O$ | 115-117 | 75 | 0.25 |
| laf | " | " | 4-$CH_2CH_3$ | $C_{18}H_{20}O$ | 108-109 | 62 | |
| lag | " | " | 4-$OCH_3$ | $C_{17}H_{18}O_2$ | 139-141 | 88 | 0.2 |
| lah | " | " | 4-Cl | $C_{16}H_{15}ClO$ | 146-148 | 76 | 0.3 |
| lai | " | " | 3,4-diCl | $C_{16}H_{14}Cl_2O$ | 145.5-147 | 58 | |
| laj | " | " | 4-F | $C_{16}H_{15}FO$ | 141.5-143 | 77 | 0.15 |
| lak | " | " | 3-$CF_3$ | $C_{17}H_{15}F_3O$ | 109-110.5 | 84 | 1.0 |
| lal | " | " | 3-$CH_3$ | $C_{17}H_{18}O$ | 115-117 | 84 | 0.2 |
| lam | " | $OCH_3$ | 4-F | $C_{16}H_{15}FO_2$ | 126.5-128 | 71 | |
| lan | " | " | 3-$CF_3$ | $C_{17}H_{15}F_3O_2$ | 72-73 | 44 | |
| lao | $CH_3CH_2$ | " | 4-$OCH_3$ | $C_{18}H_{20}O_3$ | 113-114 | 66 | |
| lap | " | " | 4-F | $C_{17}H_{17}FO_2$ | 121-122 | 49 | |
| laq | $CH_3$ | $CH_3$ | 4-OBu | $C_{20}H_{24}O_2$ | 138-139 | 53 | |
| lar | " | " | 4-OPr | $C_{19}H_{22}O_2$ | 127-128 | 81 | |
| las | " | " | x* | $C_{19}H_{22}O_3$ | 143-145 | 70 | |
| lat | $CH_3CH_2$ | $CH_3CH_2$ | 4-$OCH_3$ | $C_{19}H_{22}O_2$ | 122.5-123.5 | 55 | |
| lau | " | " | 4-$CH_3$ | $C_{19}H_{22}O$ | 113-114 | 70 | |

cont.

| Compound No. | $R_1$ | $R_2$ | X | Formula | mp(C°) | Inhibition of 5-LO % at 1 M $IC_{50}$ ( M) |
|---|---|---|---|---|---|---|
| 1av | $(CH_3)_2CH$ | $(CH_3)_2CH$ | 4-$OCH_3$ | $C_{21}H_{26}O_2$ | 83-85 | 71 |
| 1aw | " | " | 3,4-di$OCH_3$ | $C_{22}H_{28}O_3$ | 161-163 | 57 |
| 1ax | " | " | 4-F | $C_{20}H_{23}FO$ | 51-53 | 32 |
| 1ay | " | " | 3-$CF_3$ | $C_{21}H_{23}F_3O$ | 55-57.5 | 34 |
| 1az | $OCH_3$ | F | 4-$OCH_3$ | $C_{16}H_{15}FO_3$ | 151-152 | 63 |

x* = 4-$O(CH_2)_2OCH_3$

Inhibition of Antigen-Induced, SRS-A Mediated Bronchioconstriction in Conscious Guinea Pigs

This model is used to measure the ability of a drug to inhibit 5-lipoxygenase *in vivo*. SRS-A (slow reacting substance of anaphylaxis - also referred to as leukotrienes $C_4$, $D_4$ and $E_4$) is released by cells in

the sensitized guinea pig upon stimulation by antigen, resulting in bronchioconstriction and change in pulmonary function. The leukotrienes are biosynthesized from arachidonic acid by the enzyme 5-lipoxygenase. Therefore, an inhibitor of 5-lipoxygenase could reduce the production of SRS-A and attenuate the bronchioconstriction.

Male outbred Hartley strain albino guinea pigs (250-300 grams) were sensitized by i.p. injection of ovalbumin (3mg/kg) followed by an i.p. injection of Bordetella pertussis (about $5x10^9$ killed organisms) and studied 14 days later.

Sensitized guinea pigs (fasted for 18 hours) were anesthesized and a catheter-tip pressure transducer was inserted into the pleural space of each animal. The animals are pretreated (i.p.) with pyrilamine (10mg/kg) and indomethacin (10mg/kg) to antagonize the effects of histamine and inhibit cyclooxygenase respectively. Cyclooxygenase products and histamine are released endogenously along with SRS-A, upon antigen challenge. Test compound is also administered i.p., 60 minutes before antigen challenge.

The guinea pigs were placed in a "head out" plethysmograph and allowed to recover from anesthesia. Airflow into the respiratory system, pleural pressure and airflow signals were measured, and tidal volume, dynamic pulmonary compliance ($C_{dyn}$) and breathing frequency were calculated according to a published technique (E.G. Damen et al., Toxicol. Appl. Pharmacol., 1982, 64, 465). After baseline values for pulmonary function were obtained, the animals were challenged by aerosolized ovalbumin. The overall changes in pulmonary function parameters were determined by calculating the mean changes from baseline during the period 5 to 15 minutes following antigen challenge.

Drug efficacy was determined by comparing the mean of the percent decrease in $C_{dyn}$ from baseline in drug treated animals (N = 4-6) to control animals (N = 10-12).

Table 2 shows the effect of several of the compounds from Table 1 in the antigen-induced, SRS-A mediated bronchioconstriction model.

Table 2. Inhibition of Antigen-Induced SRS-A Mediated Bronchioconstriction in Conscious Guinea Pigs.

TABLE 2

| Compound No. [1,2] | % Inhibition of Bronchioconstriction[3] |
|---|---|
| 1a | 75 |
| 1d | 41 |
| 1e | 51 |
| 1f | 57 |
| 1o | 26 |
| 1x | 39 |
| 1ab | 34 |
| 1ad | 53 |
| 1ae | 59 |
| 1ag | 28 |
| 1aj | 43 |
| 1al | 49 |

1. Compound number refers to the compound numbers found in Table 1.

2. Compounds were administered i.p. in a 5% Tween® 80 suspension, at a dose of 30mg/kg.

3. % inhibition of the decrease in $C_{dyn}$ seen in antigen challenged animals.

Inhibition of Antigen-Induced, Late Phase Inflammatory Cell Infiltration in Guinea Pigs

This model is used to measure the ability of a drug to inhibit 5-lipoxygenase in vivo. In addition to SRS-A, a chemotactic substance $LTB_4$ is also released by cells in the sensitized guinea pig lung upon stimulation by antigen. As a result there is a measurable influx of cells into the lung. Therefore, an inhibitor

of 5-lipoxygenase could reduce the formation of $LTB_4$ and thereby reduce the cell influx into the lung.

Male outbred Hartley strain albino guinea pigs (250-300 grams) were sensitized to ovalbumin as in the procedure for inhibition of antigen-induced, SRS-A mediated bronchioconstriction in conscious guinea pigs.

Sensitized guinea pigs were fasted and pretreated with pyrilamine and indomethacin as in bronchioconstriction testing procedure. Test compounds, suspended in 1% acacia, were administered 60 minutes before antigen challenge. Four hours after challenge with aerosolized ovalbumin, the animals were anesthetized, and exsanguinated by severing of the abdominal artery and inferior vena cava. Whole lung lavage was performed with three 5 mL aliquots of normal saline buffered with sodium bicarbonate.

Total cell counts were performed on a Coulter Counter, and differential cell counts were performed. Compounds were evaluated for their ability to alter the antigen-induced total cell influx and, more specifically, neutrophil (PMN) influx.

Table 3. Inhibition of Antigen-Induced, Late Phase Inflammatory Cell Infiltration in Guinea Pigs.

TABLE 3

| Compound No.[1] | Dose(mg/kg) | Route | % Inh. Total Cells | % Inh. PMN |
|---|---|---|---|---|
| 1a | 30 | ip | 78 | 95 |
| 1a | 30 | po | 51 | 14 |
| 1f | 30 | ip | 77 | 54 |
| 1h | 10 | ip | 55 | 46 |
| 1i | 30 | ip | 51 | 74 |
| 1i | 30 | po | 65 | 51 |
| 2a | 10 | po | 89 | 93 |

1. Compound No. refers to the compound numbers found in Table 1 except for 2a which refers to a prodrug wherein $R_1$ and $R_2$ are $CH_3$; Ar is 2-thienyl; and X is $-CH_2CH_2$.

Ex Vivo Inhibition of A23187-Induced $LTB_4$ Generation in Squirrel Monkeys.

Incubation of squirrel monkey whole blood with calcium ionophore A23187 stimulates the production of $LTB_4$. This $LTB_4$ production can be inhibited by a lipoxygenase inhibitor in vitro by incubating the blood with the inhibitor prior to A23187 challenge. By dosing squirrel monkeys with a lipoxygenase inhibitor prior to withdrawal of a blood sample, one can determine the ability of the lipoxygenase inhibitor to be absorbed into the bloodstream by measuring the inhibition of A23187 induced $LTB_4$ production ex vivo.

Squirrel monkeys (400-650g) were fasted for 18 hours, anesthetized and dosed either orally or intraperitoneally with test compound. After the desired time period, blood was withdrawn from the femoral

vein. The blood (0.5 mL) was heparinized, preincubated at 37 °C for 5 minutes and stimulated with A23187 in DMSO to give a final concentration of 30 microM. The reaction was terminated with EGTA (final concentration 10 microM), and the blood centrifuged. Levels of $LTB_4$ in the plasma were then determined by radioimmunoassay.

The results shown in Table 4 illustrate the ability of a prodrug of type 2a or 2b to be absorbed after ip or po dosing compared to the parent compound 1a.

Table 4. *Ex Vivo* Inhibition of A23187-Induced $LTB_4$ Generation in Squirrel Monkey Blood.

TABLE 4

| Compound No.[1] | Dose (mg/kg) | Route | Time After Dosing (min.) | Percent Inh. Monkey #1 | 2 | 3 |
|---|---|---|---|---|---|---|
| 1a | 30 | ip[2] | 60 | 47 | 12 | 4 |
| 1a | 30 | po[2] | 60 | 18 | 0 | 0 |
| 2a | 30 | ip[3] | 60 | 62 | 83 | 89 |
| 2a | 30 | po[3] | 60 | 50 | 71 | 66 |
| 2a | 30 | po[3] | 120 | 41 | 100 | 61 |
| 2a | 30 | po[3] | 240 | 43 | 65 | 100 |
| 2b | 30 | ip[4] | 60 | 78 | 56 | 69 |
| 2b | 30 | po[4] | 60 | 62 | 54 | 33[5] |

1. Compound No. 1a refers to Compound 1a in Table 1. 2a refers to a prodrug wherein $R_1$ and $R_2$ are $CH_3$, Ar is 2-thienyl and X is $CH_2CH_2$-. 2b refers to a prodrug wherein $R_1$ and $R_2$ are $CH_3$, Ar is 2-thienyl, $R_5$, $R_6$ and $R_7$ are H and n=1, hydrochloride salt.

2. Suspended in 1% acacia and administered at 6 mL/kg.

3. Dissolved in dilute alkali and administered at 6 mL/kg.

4. Dissolved in distilled water and administered at 6 mL/kg.

5. Administered at 24 mg/kg instead of 30 mg/kg.

Additionally, the following compounds given by way of Example, also within the scope of the present invention, may be made with techniques known of those skilled in the art.

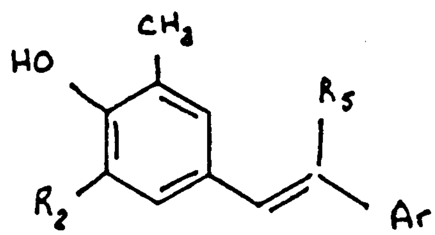

| R2 | R5 | Ar |
|---|---|---|
| $CH_3$ | $CO_2Et$ | Ph |
| $CH_3$ | H | 2-napthyl |
| $CH_3$ | H | 3-thianapthenyl |
| $CH_3$ | H | 1-napthyl |
| $CH_3$ | H | 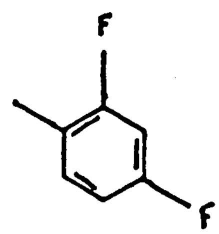 |
| $CH_3$ | H | 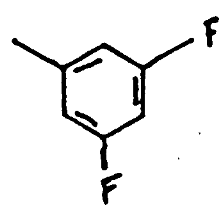 |
| $CH_3$ | $CH_3$ | 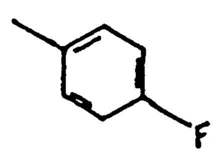 |
| $CH_3$ | n-Bu | 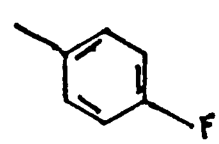 |

| | | |
|---|---|---|
| $CH_3$ | H | 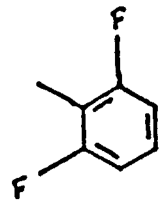 |
| $CH_3$ | H | 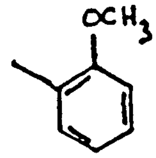 |
| $CH_3$ | H | 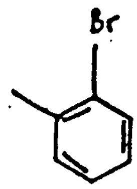 |
| $CH_3$ | H | 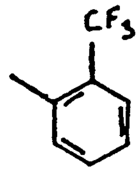 |
| $CH_3$ | H | 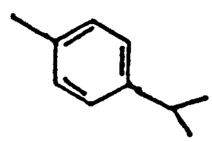 |
| $CH_3$ | H | 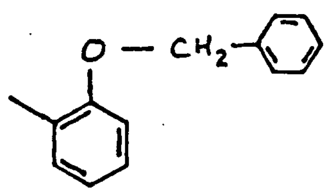 |
| $CH_3$ | H | 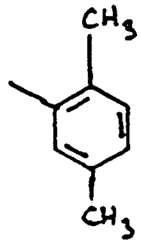 |

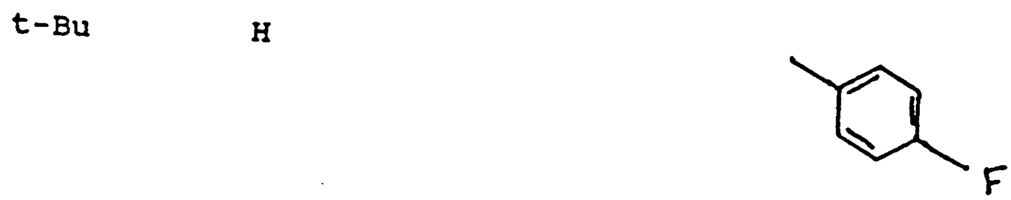

## Claims

**1.** Compounds having the general formula I

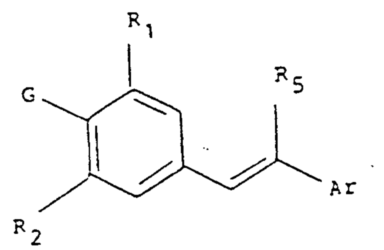

I

wherein
$R_1$ and $R_2$ are each independently branched or straight chained alkyl of 1 to 8 carbon atoms, allyl, alkoxy of 1 to 8 carbon atoms e.g. methoxy or halogen (i.e. chlorine, bromine or fluorine),$R_5$ is hydrogen, alkyl of 1 to 8 carbon atoms or $-CO_2R_8$, wherein $R_8$ is hydrogen or $C_1$-$C_3$ alkyl
G is

a) -OH

b) the group $R_3CO_2$- wherein $R_3$ is (i) $HO_2C$-X wherein X is an optionally substituted hydrocarbon chain with or without heteroatoms, or (ii) the group

$$(R_{10})(R_{11})N(\underset{R_{12}}{CH})_n$$

wherein $R_{10}$ and $R_{11}$ are hydrogen or alkyl, $R_{12}$ is hydrogen, alkyl or aryl possibly containing heteroatoms or $R_{11}$ and $R_{12}$ together form a ring, or

c) the group $HCO_2$- provided $R_5$ is $-CO_2R_8$ and $R_8$ is hydrogen or $C_1$-$C_3$ alkyl, and

Ar is an optionally substituted heteroaromatic containing 5 to 12 ring members of which up to 3 may be heteroatoms chosen from N, S or O and which may contain two fused rings or optionally substituted aromatic ring containing five to 12 carbon atoms and which may contain two fused rings, the optional substituents on the group Ar are up to three groups which may be the same or different and are chosen from $C_1$-$C_5$ alkoxy, halogen (i.e. chlorine, bromine or fluorine),trifluoromethyl, alkyl of 1 to 8 carbon atoms and arylalkoxy with the proviso that Ar is not

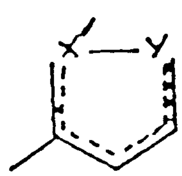

wherein --- is single or double bond and X' and Y are
(i) N

(ii) $NR_4$, where $R_4$ is H, alkyl of 1 to 5 carbon atoms,

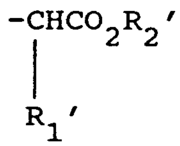

where $R_1$' and $R_2$' may be the same or different and are H or alkyl of 1 to 5 carbon atoms, $C(O)R_4$' wherein $R_4$' is hydrogen or alkyl of 1 to 5 carbon atoms, cycloalkyl of from three to twenty carbons having from three to eight ring carbons, aryl or aralkyl;
(iii) O; or
(iv) S;
and the further proviso that Ar is not

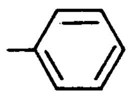
or
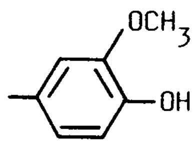

when $R_1$ and $R_2$ are both methyl,
and the further proviso that
Ar is not p-pyridyl when G is hydroxy, $R_1$ is methyl, iso-propyl or tert. butyl and $R_2$ is iso-propyl, tert. butyl or cyclohexyl and $R_5$ is hydrogen or methyl;
and the further proviso that Ar is not o-pyridyl when G is hydroxy, $R_1$ is methyl, iso-propyl or tert. butyl and $R_2$ is iso-propyl or tert. butyl, and $R_5$ is hydrogen; or another pharmaceutically acceptable ester or pro-drug thereof or salt or acid addition salt of the foregoing compounds.

**2.** A compound as recited in Claim 1 wherein Ar is 2- or 3-thienyl.

**3.** A compound as recited in Claim 1 wherein Ar is 2-thienyl.

**4.** A compound as recited in Claim 1 wherein Ar is 1-napthyl or 2-napthyl.

**5.** A compound as recited in Claim 1 wherein Ar is 3-thianapthenyl.

**6.** A compound as recited in Claim 1 wherein Ar is phenyl optionally mono- or poly-substituted by halogen, alkyl, alkoxy or trifluoromethyl.

**7.** A compound according to in Claim 1, this compound being

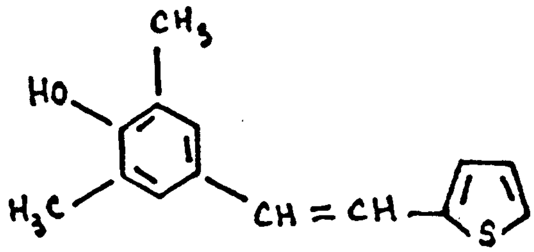

,

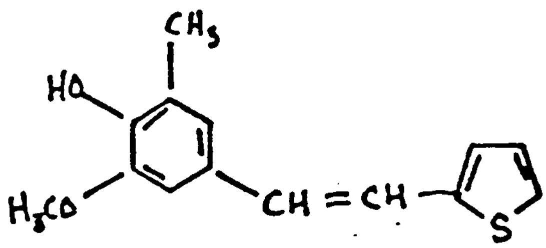

,

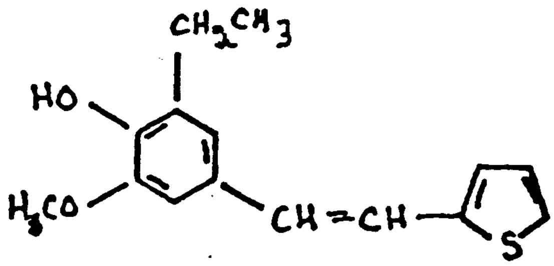

,

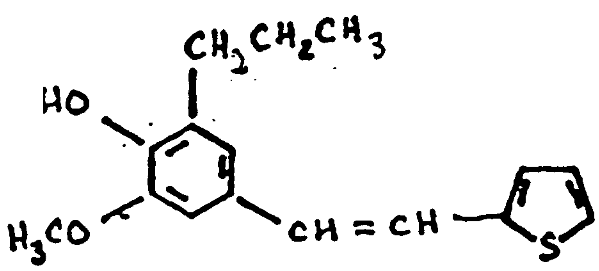

,

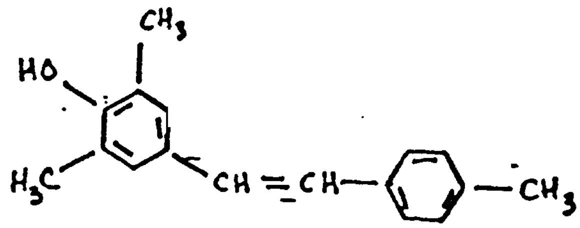

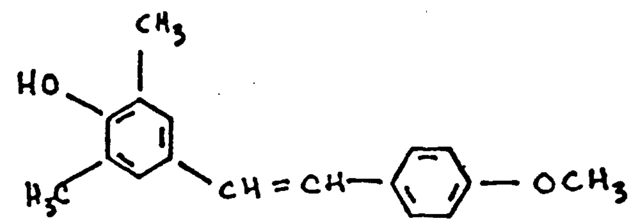

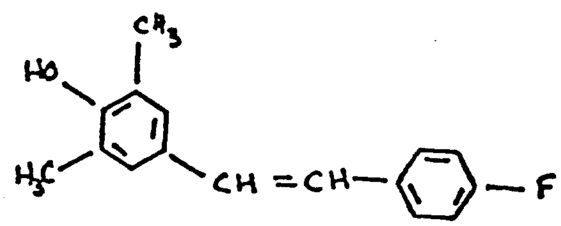

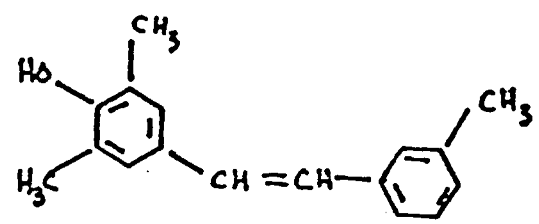

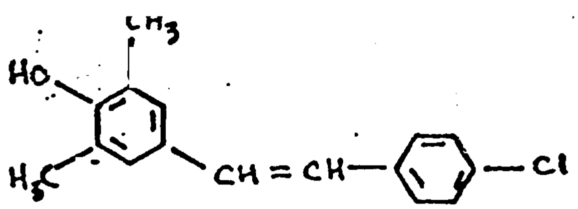

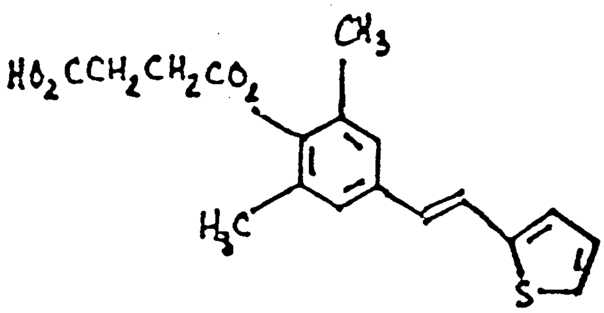

or

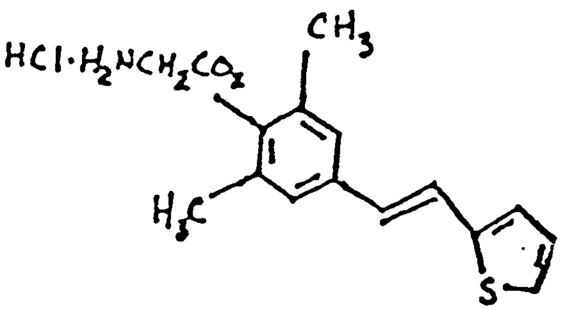

**8.** Compounds as recited in Claim 1 having the following formula:

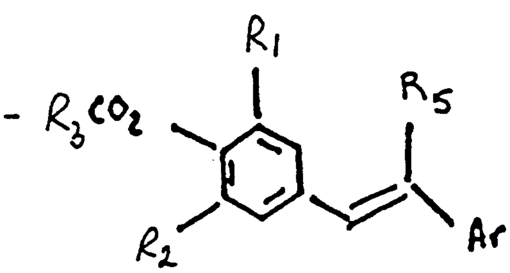

wherein $R_3$ is $HO_2C$-X, where X is an optionally substituted hydrocarbon chain with or without heteroatoms and
$R_5$ is hydrogen or $C_1$-$C_5$ alkyl, or $CO_2R_8$, wherein
$R_8$ is hydrogen or $C_1$-$C_3$ alkyl, or other ester thereof, or any salt thereof.

**9.** Compounds as defined in claim 1, having the formula

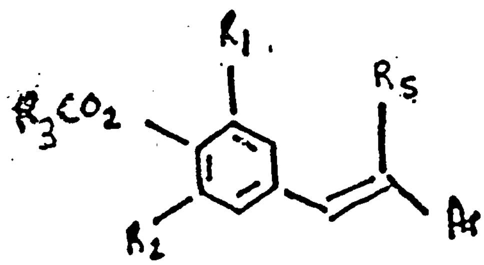

wherein $R_3$ is

$$(R_{10})\ (R_{11})\ N\ (\underset{R_{12}}{CH})_n,$$

where $R_{10}$ and $R_{11}$ are each independently hydrogen or alkyl, $R_{12}$ is hydrogen, alkyl or aryl, with or without heteroatoms or $R_{11}$ and $R_{12}$ form a ring, and n is an integer from one to five, and $R_5$ is hydrogen, or a salt thereof.

**10.** Compounds as recited in Claim 1 having the following formula:

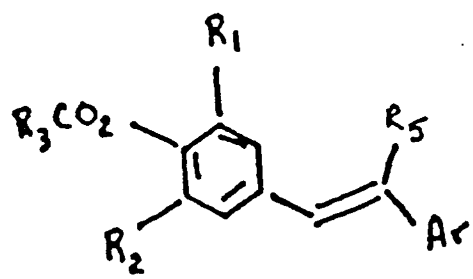

wherein $R_3$ is $HO_2C$-X, where X is an optionally substituted hydrocarbon chain with or without heteroatoms, or $R_3$ is hydrogen, or $R_3$ is

$$(R_{10}),\ R_{11}),\ N\ (\underset{R_{12}}{CH})_n,$$

where $R_{10}$ and $R_{11}$ are each independently hydrogen or alkyl, $R_{12}$ is hydrogen, alkyl or aryl, with or without heteroatoms or $R_{11}$ and $R_{12}$ form a ring, and n is an integer from one to five, and $R_5$ is $CO_2R_8$, where $R_8$ is hydrogen or alkyl or other ester thereof, or a salt thereof.

**11.** A compound as defined in any one of the foregoing claims for use in the treating a disease in a warm blooded animal by inhibiting the enzyme 5-lipoxygenase.

**12.** A pharmaceutical composition which comprises a compound as defined in any one of claims 1 to 11 and a pharmaceutically acceptable carrier or excepient.

**13.** A method of producing a compound as defined in any one of claims 1 to 11 which comprises

a) to prepare a compound of formula 1 wherein $R_5$ is hydrogen and G is -OH, reacting a compound of formula 7

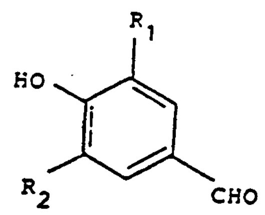

7

with an aryl or heteroaryl acetic acid 8

$ArCH_2CO_2H$ 8

to yield a compound of formula 1" having the structure

1"

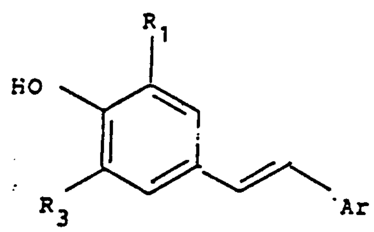

wherein Ar, $R_1$ and $R_2$ are as defined above,
b) to prepare a compound of formula 1, wherein $R_5$ is $CO_2R_8$ and G is -OH, reacting a compound of formula 7 as defined above with an aryl or heteroaryl acetic acid ester having the formula 9

$ArCH_2CO_2R_8$ 9

to yield a compound having formula 10

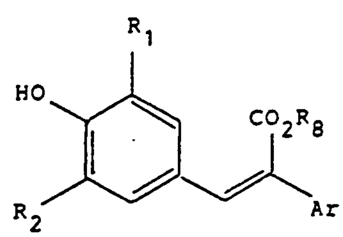

10

wherein Ar, $R_1$, $R_2$ are as defined above and $R_8$ is $C_1$-$C_3$ alkyl,
c) to provide a compound of formula 1 wherein $R_8$ is hydrogen, hydrolysing an ester of formula 10'

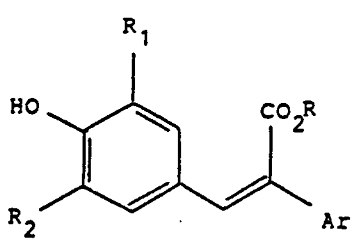

10'

wherein R, which may be $R_8$ when this is $C_1$-$C_3$ alkyl, is an ester forming group capable of being removed in a manner known per se, to yield the free acid 11

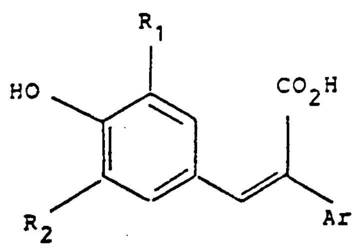

11

wherein Ar, $R_1$ and $R_2$ are as defined above:

d) to provide a compound of formula 1 wherein $R_5$ is hydrogen, decarboxylation of an acid of formula 11 to yield a compound of formula 1"

e) to yield a compound of formula 1 wherein $R_5$ is $C_1$-$C_5$ alkyl, deprotecting a compound having the formula 12

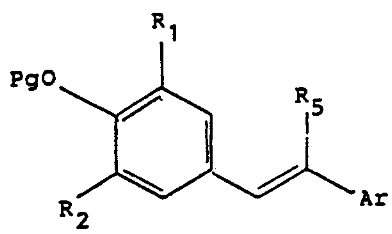

12

wherein $R_1$, $R_2$ and Ar are as defined above and Pg is a hydroxy protecting group

f) to yield a compound of formula 13

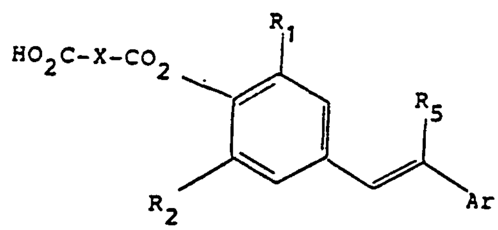

13

reacting a compound of the formula 1 wherein G is OH with a compound of the formula

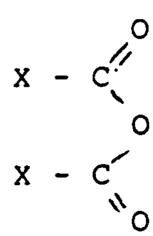

g) to prepare a compound of formula 1 wherein G is the group

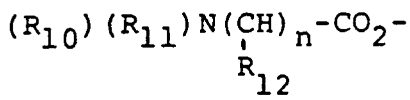

15

as defined above,
reacting a compound of the formula 1 wherein G is OH and $R_5$ is alkyl or a compound of formula 10' as defined above with a compound of the formula

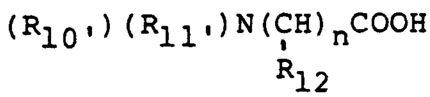

16

wherein $R_1$, $R_2$ and $R_5$ are as defined above, $R_{10'}$ and $R_{11'}$ are hydrogen or alkyl with the proviso that if one is hydrogen the other is alkyl or a removable protecting group, and $R_{12}$ is hydrogen, alkyl or aryl which may contain at least one heteroatom, and wherein $R_{11'}$ and $R_{12}$ may together form a ring, and n is an integer from one to five, and thereafter removing the groups $R_{10'}$ and/or $R_{11'}$ when these are protecting groups and if necessary or desired removing the group R;

and thereafter, if desired, treating the product of process a) to g) by one or more of the following finishing steps in any desired order:

i) removing an ester group $R_8$ to yield a free acid

ii) esterifying a free acid to yield a pharmaceutically acceptable ester wherein the ester group is capable of being cleaved in vivo to yield the free acid

iii) forming a pharmaceutically acceptable salt of the free acid e.g. the salt of an alkali metal or alkaline earth metal e.g. sodium, potassium or calcium

iv) forming an acid addition salt of a compound of formula 1 in particular such a compound wherein $R_3$ is the group

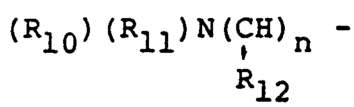

**14.** Use of a compound as defined in any one of claims 1 to 11 for the preparation of a pharmaceutical composition for the treatment of inflammation and in diseases where products of 5-lipoxygenase metabolism play a role, such as asthma, psoriasis, rheumatoid arthritis and inflammatory bowel disease, and for inbiting the enzyme cyclooxygenase.

## Patentansprüche

**1.** Verbindungen der allgemeinen Formel I

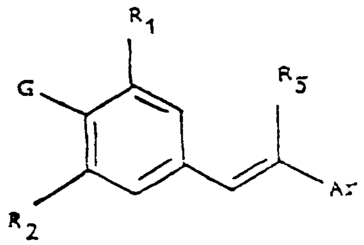

(I),

worin

$R_1$ und $R_2$ unabhängig voneinander verzweigtes oder geradkettiges Alkyl mit 1 bis 8 Kohlenstoffatomen, Allyl, Alkoxy mit 1 bis 8 Kohlenstoffatomen (z.B. Methoxy) oder Halogen (z.B. Chlor, Brom oder Fluor);

$R_5$ Wasserstoff, Alkyl mit 1 bis 8 Kohlenstoffatomen oder $-CO_2R_8$, worin $R_8$ Wasserstoff oder $C_1$-$C_3$-Alkyl darstellt;

G

a) -OH

b) die Gruppe $R_3CO_2$-, worin $R_3$

(i) $HO_2C$-X darstellt, wobei X einer gegebenenfalls substituierten Kohlenwasserstoffkette mit oder ohne Heteroatome entspricht, oder

(ii) die Gruppe

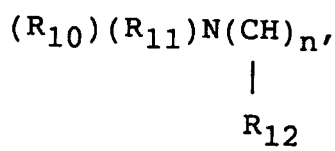

worin $R_{10}$ und $R_{11}$ Wasserstoff oder Alkyl und $R_{12}$ Wasserstoff, Alkyl oder gegebenenfalls Heteroatome enthaltendes Aryl darstellen oder $R_{11}$ und $R_{12}$ zusammen einen Ring bilden, oder

c) die Gruppe $HCO_2$-, vorausgesetzt, dass $R_5$ für $-CO_2R_8$ steht und $R_8$ Wasserstoff oder $C_1$-$C_3$-Alkyl ist; und

Ar einen gegebenenfalls substituierten 5- bis 12-gliedrigen heteroaromatischen Ring mit bis zu 3 Heteroatomen aus der Gruppe N, S oder O, wobei der heteroaromatische Ring zwei kondensierte Ringe enthalten kann, oder einen gegebenenfalls substituierten aromatischen Ring mit 5 bis 12 Kohlenstoffatomen, der zwei kondensierte Ringe enthalten kann, die Zahl der gegebenenfalls vorhandenen Substituenten der Gruppe Ar bis zu drei betragen kann, wobei diese gleich oder verschieden sein können und aus der aus $C_1$-$C_5$-Alkoxy, Halogen (z.B. Chlor, Brom oder Fluor), Trifluormethyl, Alkyl mit 1 bis 8 Kohlenstoffatomen und Arylalkoxy bestehenden Gruppe ausgewählt sind,

bedeuten, vorausgesetzt, dass Ar nicht für

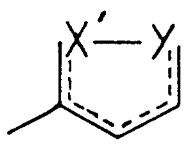

,

steht, worin --- einer Einfach- oder Doppelbindung entspricht und X' und Y, wie folgt definiert sind:

(i) N

(ii) $NR_4$, worin $R_4$ für Wasserstoff, Alkyl mit 1 bis 5 Kohlenstoffatomen,

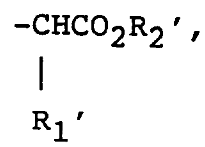

worin $R_1$' und $R_2$', die gleich oder verschieden sein können, Wasserstoff oder Alkyl mit 1 bis 5 Kohlenstoffatomen darstellen,
$C(O)R_4$', worin $R_4$' Wasserstoff oder Alkyl mit 1 bis 5 Kohlenstoffatomen, Cycloalkyl mit drei bis zwanzig Kohlenstoffatomen, wobei drei bis acht Ringkohlenstoffatome vorhanden sind, Aryl oder Aralkyl darstellt;
(iii)O; oder
(iv) S;

ferner vorausgesetzt, dass Ar nicht

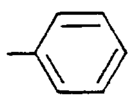

oder

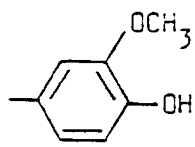

bedeutet, wenn $R_1$ und $R_2$ beide für Methyl stehen;
weiterhin vorausgesetzt, dass Ar nicht p-Pyridyl darstellt, wenn G für Hydroxy, $R_1$ für Methyl, Isopropyl oder tert.-Butyl und $R_2$ für Isopropyl, tert.-Butyl oder Cyclohexyl stehen und $R_5$ Wasserstoff oder Methyl bedeutet; und
weiterhin vorausgesetzt, dass Ar nicht o-Pyridyl darstellt, wenn G für Hydroxy, $R_1$ für Methyl, Isopropyl oder tert.-Butyl und $R_2$ für Isopropyl oder tert.-Butyl stehen und $R_5$ Wasserstoff bedeutet,
sowie andere pharmazeutisch annehmbare Ester, Promedikamente oder Salze oder Säureadditionssalze der vorstehend genannten Verbindungen.

2. Verbindung nach Anspruch 1, worin Ar für 2- oder 3-Thienyl steht.

3. Verbindung nach Anspruch 1, worin Ar für 2-Thienyl steht.

4. Verbindung nach Anspruch 1, worin Ar für 1-Naphthyl oder 2-Naphthyl steht.

5. Verbindung nach Anspruch 1, worin Ar für 3-Thianaphthenyl steht.

6. Verbindung nach Anspruch 1, worin Ar für gegebenenfalls durch Halogen, Alkyl, Alkoxy oder Trifluormethyl mono- oder polysubstituiertes Phenyl steht.

7. Verbindung nach Anspruch 1, dadurch gekennzeichnet, dass sie einer der folgenden Formeln entspricht:

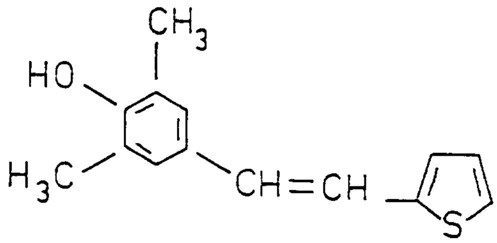

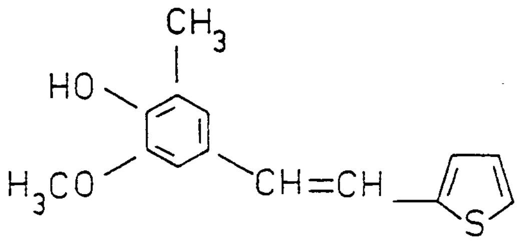

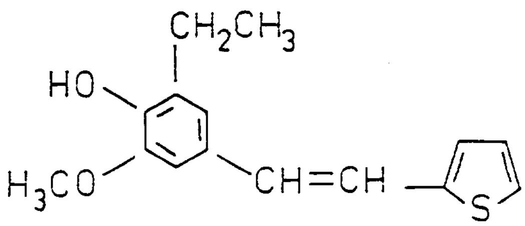

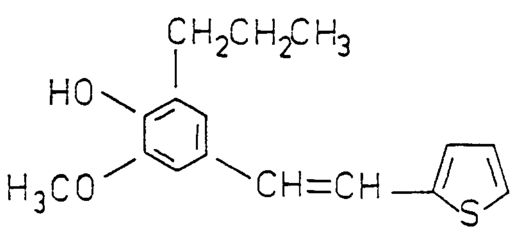

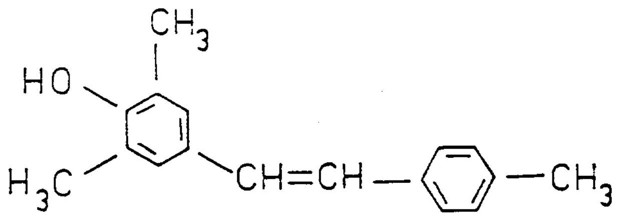

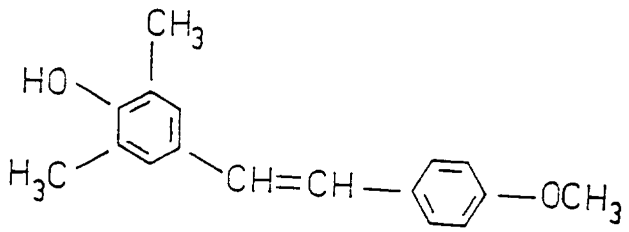

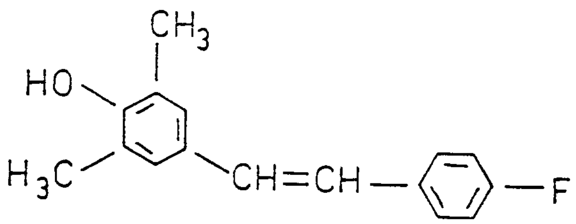

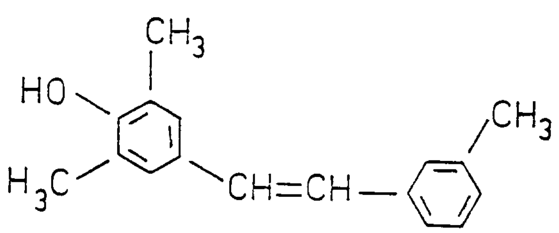

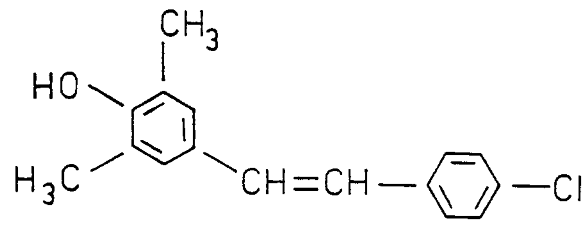

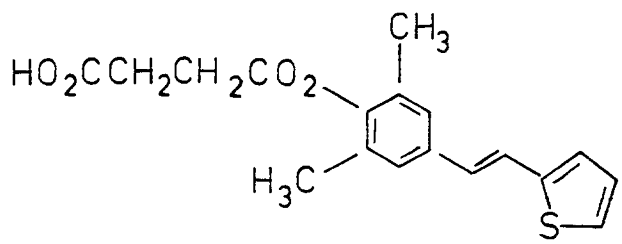

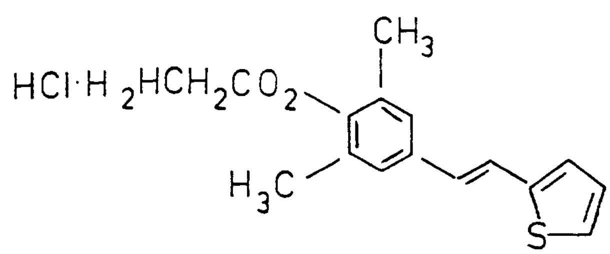

**8.** Verbindungen nach Anspruch 1, welche die folgende Formel

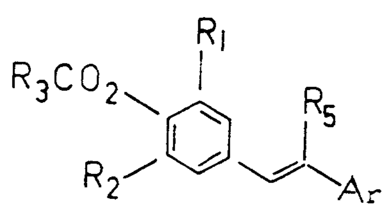

aufweisen, worin

$R_3$ $HO_2C$-X, worin X eine gegebenenfalls substituierte Kohlenwasserstoffkette mit oder ohne Heteroatome darstellt;

$R_5$ Wasserstoff oder $C_1$-$C_5$ Alkyl oder $CO_2R_8$, worin $R_8$ Wasserstoff oder $C_1$-$C_3$ Alkyl darstellt,

bedeuten, sowie deren andere Ester oder irgendein Salz davon.

**9.** Verbindungen nach Anspruch 1, welche die folgende Formel

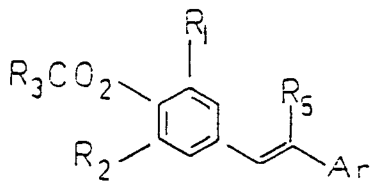

aufweisen, worin

$R_3$

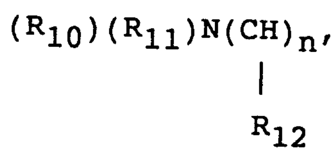

worin $R_{10}$ und $R_{11}$ unabhängig voneinander Wasserstoff oder Alkyl sind, $R_{12}$ Wasserstoff, gegebenenfalls Heteroatome aufweisendes Alkyl oder Aryl darstellen oder $R_{11}$ und $R_{12}$ einen Ring bilden und n für eine ganze Zahl von 1 bis 5 steht; und

$R_5$ Wasserstoff

bedeuten, oder ein Salz dieser Verbindung.

**10.** Verbindungen nach Anspruch 1, welche die folgende Formel

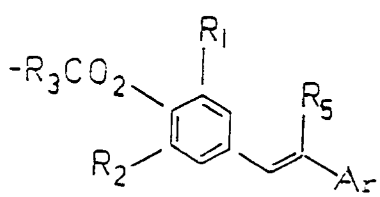

aufweisen, worin

$R_3$ $HO_2C$-X, worin X eine gegebenenfalls substituierte Kohlenwasserstoffkette mit oder ohne Heteroatome darstellt, oder

$R_3$ Wasserstoff, oder

$R_3$

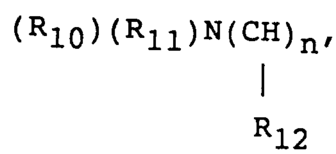

worin $R_{10}$ und $R_{11}$ unabhängig voneinander Wasserstoff oder Alkyl sind, $R_{12}$ Wasserstoff, gegebenenfalls Heteroatome aufweisendes Alkyl oder Aryl darstellt oder $R_{11}$ und $R_{12}$ einen Ring bilden und n für eine ganze Zahl von 1 bis 5 steht; und

$R_5$ $CO_2R_8$, worin $R_8$ Wasserstoff oder Alkyl darstellt,

bedeuten, sowie anderen Ester oder Salze davon.

**11.** Verbindung nach einem der vorstehenden Ansprüche zur Verwendung für die Behandlung einer Krankheit bei einem warmblütigen Tier durch Inhibierung des Enzyms 5-Lipoxygenase.

**12.** Pharmazeutische Zubereitung, enthaltend eine Verbindung nach einem der Ansprüche 1 bis 11 und eine pharmazeutisch annehmbare Trägersubstanz oder ein Corrigens.

**13.** Verfahren zur Herstellung einer Verbindung nach einem der Ansprüche 1 bis 11, welches die folgenden Verfahrensschritte umfasst: dass man

a) zur Herstellung einer Verbindung der Formel 1, worin $R_5$ Wasserstoff und G -OH bedeuten, eine Verbindung der Formel 7

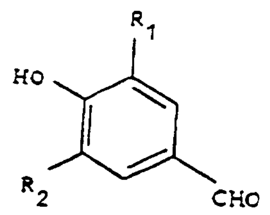

7

mit einer Aryl- oder Heteroarylessigsäure der Formel 8

$ArCH_2CO_2H$ 8

umsetzt, um zu einer Verbindung der Formel 1", welche die folgende Struktur aufweist,

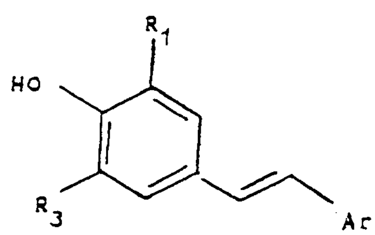

1",

worin Ar, $R_1$ und $R_2$ wie oben definiert sind, zu gelangen;

b) zur Herstellung einer Verbindung der Formel 1, worin $R_5$ $CO_2R_8$ und G -OH bedeuten, eine Verbindung der Formel 7, wie oben definiert, mit einem Aryl- oder Heteroarylessigsäureester der Formel 9

$ArCH_2CO_2R_8$ 9

umsetzt, um zu einer Verbindung der Formel 10

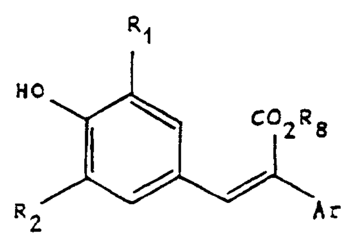

10,

worin Ar, $R_1$, $R_2$ wie oben definiert sind und $R_8$ $C_1$-$C_3$-Alkyl bedeutet, zu gelangen;

c) zur Herstellung einer Verbindung der Formel 1, worin $R_8$ Wasserstoff bedeutet, einen Ester der Formel 10'

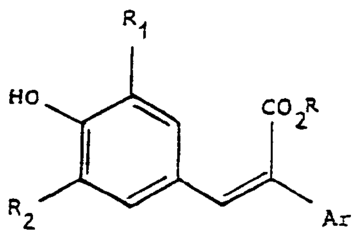

10',

worin R, das für $R_8$ stehen kann, wenn dieses $C_1$-$C_3$-Alkyl darstellt, eine esterbildende Gruppe, die auf an sich bekannte Weise entfernt werden kann, bedeutet, hydrolysiert, um zu der freien Säure 11

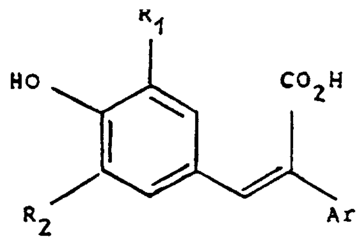

11,

worin Ar, $R_1$ und $R_2$ wie oben definiert sind, zu gelangen;

d) zur Herstellung einer Verbindung der Formel 1, worin $R_5$ Wasserstoff bedeutet, eine Säure der Formel 11 decarboxyliert, um zu einer Verbindung der Formel 1" zu gelangen;

e) zur Herstellung einer Verbindung der Formel 1, worin $R_5$ für $C_1$-$C_5$-Alkyl steht, bei einer Verbindung der Formel 12

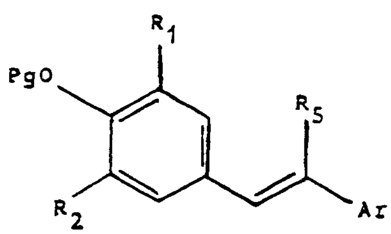

12,

worin $R_1$, $R_2$ und Ar wie oben definiert sind und pg für eine Hydroxyschutzgruppe steht, die Schutzgruppe entfernt;

f) zur Herstellung einer Verbindung der Formel 13

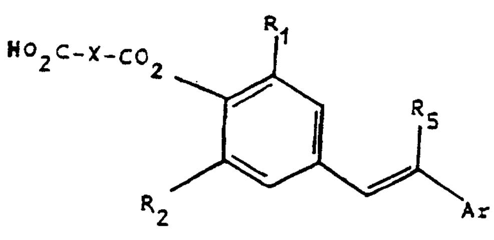

13

eine Verbindung der Formel 1, worin G für OH steht, mit einer Verbindung der Formel

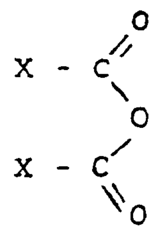

umsetzt;

g) zur Herstellung einer Verbindung der Formel 1, worin G der Gruppe

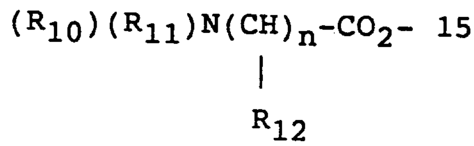

15,

wie oben definiert, entspricht, eine Verbindung der Formel 1, worin G für OH und $R_5$ für Alkyl stehen, oder einer Verbindung der Formel 10', wie oben definiert, mit einer Verbindung der Formel 16

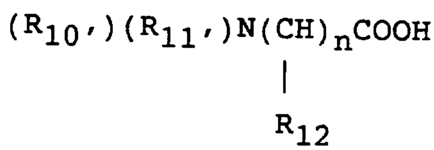

16,

worin $R_1$, $R_2$ und $R_5$ wie oben definiert sind, $R_{10'}$ und $R_{11'}$ für Wasserstoff oder Alkyl stehen, mit der Massgabe, dass, wenn einer dieser Reste für Wasserstoff steht, der andere Alkyl oder eine entfernbare Schutzgruppe darstellt, und $R_{12}$ Wasserstoff, Alkyl oder Aryl, welches mindestens ein Heteroatom enthalten kann, bedeutet und worin $R_{11}$ und $R_{12}$ zusammen einen Ring bilden können und n für eine ganze Zahl von eins bis fünf steht, umsetzt und danach die Gruppen $R_{10'}$ und/oder $R_{11'}$, wenn diese Schutzgruppen darstellen, entfernt und, sofern notwendig oder erwünscht, die Gruppe R abspaltet; und

danach, sofern erwünscht, das Produkt aus den Verfahrensschritten a) bis g) einem oder mehreren der nachfolgend aufgeführten abschliessenden Schritte in beliebiger Reihenfolge unterwirft:

i) Entfernen einer Estergruppe $R_8$ zur Gewinnung einer freien Säure;

ii) Veresterung einer freien Säure zur Gewinnung eines pharmazeutisch annehmbaren Esters, wobei die Estergruppe so beschaffen ist, dass sie in vivo abgespalten werden kann, um die freie Säure zu ergeben;

iii) Bildung eines pharmazeutisch annehmbaren Salzes der freien Säure, z.B. des Salzes eines Alkalimetalls oder Erdalkalimetalls, z.B. Natrium, Kalium oder Calcium;

iv) Bildung eines Säureadditionssalzes einer Verbindung von Formel 1, insbesondere einer solchen Verbindung, worin $R_3$ der Gruppe

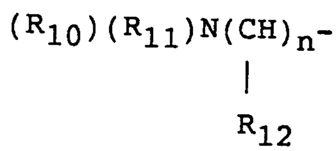

entspricht.

14. Verwendung einer Verbindung, wie in den Ansprüchen 1 bis 11 definiert, zur Herstellung von pharmazeutischen Zubereitungen zur Behandlung von Entzündungen und Krankheiten, bei denen Produkte des 5-Lipoxygenase-Metabolismus eine Rolle spielen, wie Asthma, Psoriasis, rheumatische Arthritis und entzündliche Darmerkrankungen, sowie zur Inhibition des Enzyms Cyclooxygenase.

## Revendications

1. Composés répondant à la formule générale I

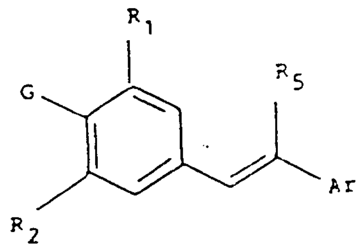

I

dans laquelle
$R_1$ et $R_2$ sont chacun indépendamment un alkyle à chaîne ramifiée ou droite de 1 à 8 atomes de carbone, un allyle, un alcoxy de 1 à 8 atomes de carbone comme méthoxy ou un halogène (c'est-à-dire le chlore, le brome ou le fluor), $R_5$ est un hydrogène, un alkyle de 1 à 8 atomes de carbone ou $-CO_2R_8$, où $R_8$ est un hydrogène ou un alkyle en $C_1$-$C_3$
G est
a) -OH
b) le groupe $R_3CO_2$-, dans lequel $R_3$ est (i) $HO_2C$-X, où X est une chaîne hydrocarbonée facultativement substituée, avec ou sans hétéro-atomes, ou (ii) le groupe

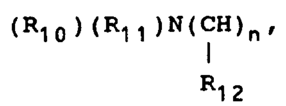

où $R_{10}$ et $R_{11}$ sont un hydrogène ou un alkyle, $R_{12}$ est un hydrogène, un alkyle ou un aryle contenant éventuellement des hétéro-atomes ou $R_{11}$ et $R_{12}$ forment ensemble un cycle, ou
c) le groupe $HCO_2$-, sous réserve que $R_5$ soit $-CO_2R_8$ et $R_8$ soit un hydrogène ou un alkyle en $C_1$-$C_3$, et
Ar est un hétéro-aromatique facultativement substitué contenant 5 à 12 termes nucléaires dont jusqu'à 3 peuvent être des hétéro-atomes choisis parmi N, S ou O et qui peut contenir deux cycles condensés ou un cycle aromatique facultativement substitué contenant 5 à 12 atomes de carbone et qui peut contenir deux cycles condensés, les substituants facultatifs sur le groupe Ar sont jusqu'à trois groupes qui peuvent être semblables ou différents et sont choisis parmi un alcoxy en $C_1$-$C_5$, un halogène (c'est-à-dire le chlore, le brome ou le fluor), un trifluorométhyle, un alkyle de 1 à 8 atomes de carbone et un arylalcoxy, sous réserve que Ar ne soit pas

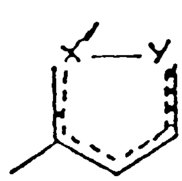

où --- est une simple ou une double liaison et X' et Y sont
(i) N

(ii) $NR_4$, où $R_4$ est H, un alkyle de 1 à 5 atomes de carbone,

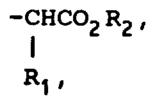

où $R_{1'}$ et $R_{2'}$ peuvent être semblables et différents et sont H ou un alkyle de 1 à 5 atomes de carbone,

$C(O)R_{4'}$, où $R_{4'}$ est un hydrogène ou un alkyle de 1 à 5 atomes de carbone, un cycloalkyle de 3 à 20 carbones ayant 3 à 8 carbones nucléaires, un aryle ou un aralkyle ;

(iii) O ; ou

(iv) S ;

et sous réserve de plus que Ar ne soit pas

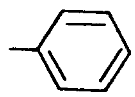 ou

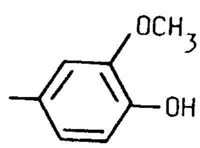

lorsque $R_1$ et $R_2$ sont tous deux un méthyle,

et de plus sous réserve que

Ar ne soit pas un p-pyridyle lorsque G est un hydroxy, $R_1$ est un méthyle, un isopropyle ou un tert-butyle et $R_2$ est un isopropyle, un tert-butyle ou un cyclohexyle et $R_5$ est un hydrogène ou un méthyle ;

et sous réserve de plus que Ar ne soit pas un o-pyridyle lorsque G est un hydroxy, $R_1$ est un méthyle, un isopropyle ou un tert-butyle et $R_2$ est un isopropyle ou un tert-butyle, et $R_5$ est un hydrogène ; ou un autre ester pharmaceutiquement acceptable ou un précurseur de médicament de ceux-ci ou un sel ou un sel d'addition d'acide des composés précédents.

**2.** Un composé selon la revendication 1, où Ar est un 2- ou un 3-thiényle.

**3.** Un composé selon la revendication 1, où Ar est un 2-thiényle.

**4.** Un composé selon la revendication 1, où Ar est un 1-napthyle ou un 2-naphtyle.

**5.** Un composé selon la revendication 1, où Ar est un 3-thianaphtényle.

**6.** Un composé selon la revendication 1, où Ar est un phényle éventuellement mono- ou polysubstitué par un halogène, un alkyle, un alcoxy ou un trifluorométhyle.

7. Un composé selon la revendication 1, ce composé étant

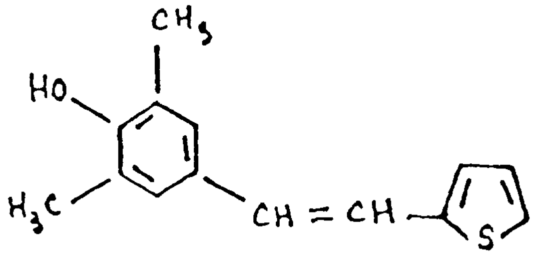

)

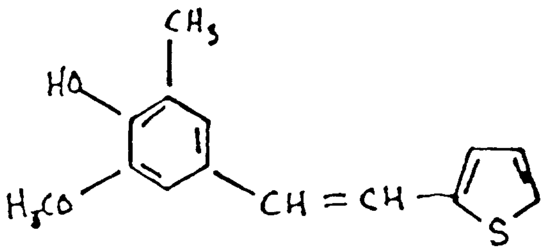

)

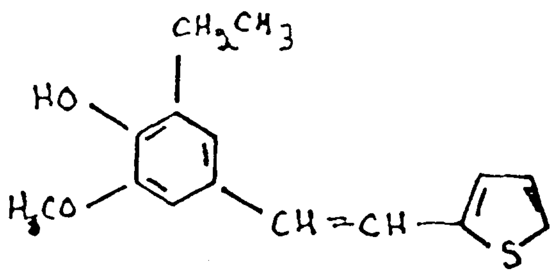

)

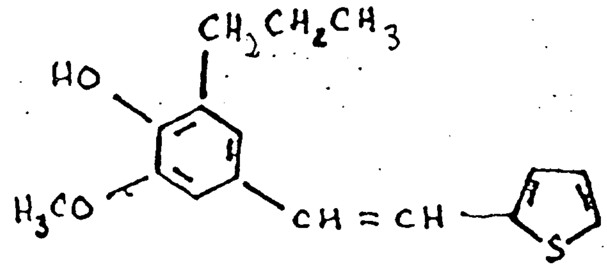

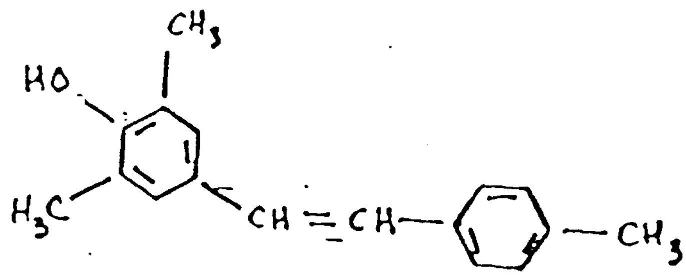

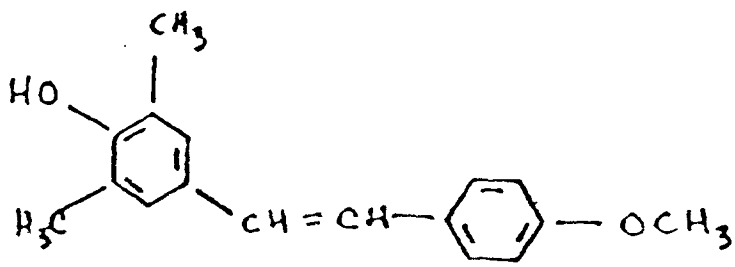

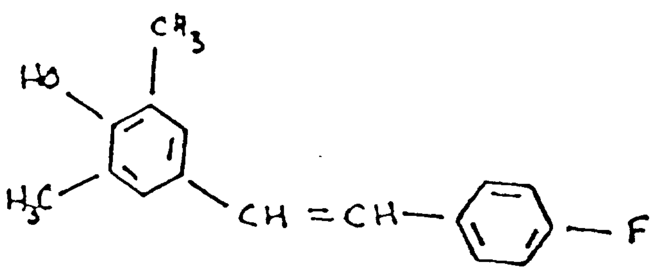

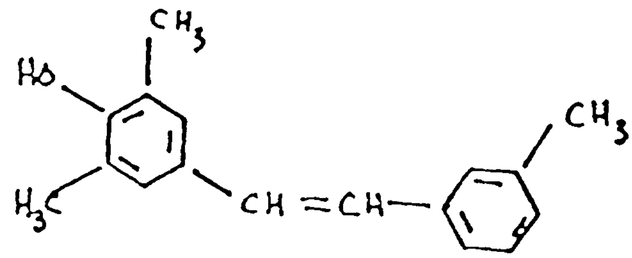

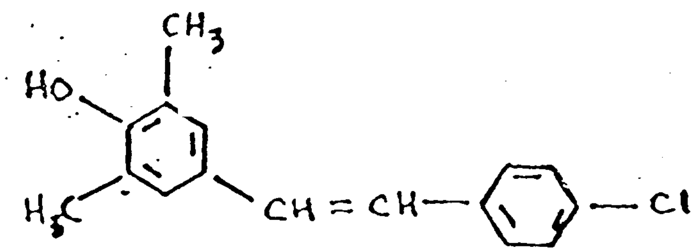

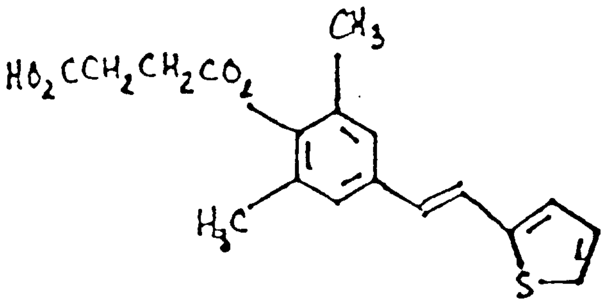

ou:

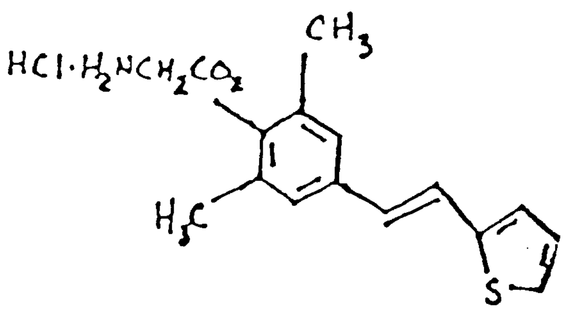

**8.** Composés selon la revendication 1 répondant à la formule suivante :

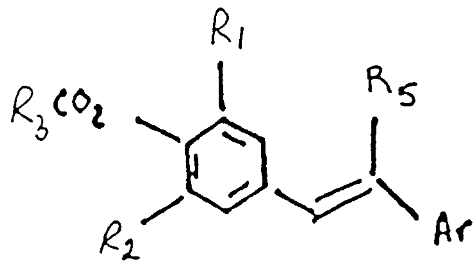

dans laquelle $R_3$ est $HO_2C$-X, où X est une chaîne hydrocarbonée facultativement substituée, avec ou sans hétéro-atomes et $R_5$ est un hydrogène ou un alkyle en $C_1$-$C_5$ ou $CO_2R_8$, où $R_8$ est un hydrogène ou un alkyle en $C_1$-$C_3$, ou un autre ester de ceux-ci ou un sel quelconque de ceux-ci.

**9.** Composés selon la revendication 1 répondant à la formule

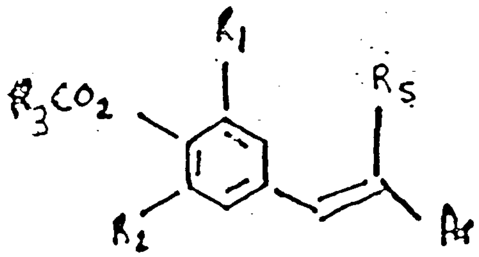

dans laquelle $R_3$ est

$$(R_{10})(R_{11})N(\underset{\displaystyle R_{12}}{\underset{|}{CH}})_n,$$

où $R_{10}$ et $R_{11}$ sont chacun indépendamment un hydrogène ou un alkyle, $R_{12}$ est un hydrogène, un alkyle ou un aryle, avec ou sans hétéro-atomes, ou $R_{11}$ et $R_{12}$ forment un cycle et n est un entier de 1 à 5, et $R_5$ est un hydrogène, ou un sel de ceux-ci.

**10.** Composés selon la revendication 1 répondant à la formule suivante :

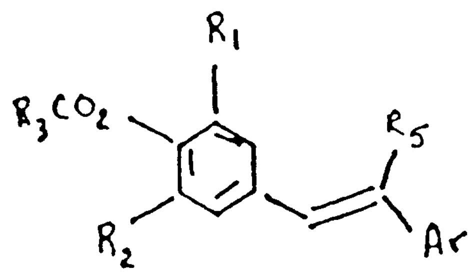

dans laquelle $R_3$ est $HO_2C$-X, où X est une chaîne hydrocarbonée facultativement substituée, avec ou sans hétéro-atomes, ou $R_3$ est un hydrogène, ou $R_3$ est

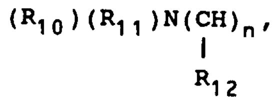

où $R_{10}$ et $R_{11}$ sont chacun indépendamment un hydrogène ou un alkyle, $R_{12}$ est un hydrogène, un alkyle ou un aryle, avec ou sans hétéro-atomes, ou $R_{11}$ et $R_{12}$ forment un cycle, et n est un entier de 1 à 5, et $R_5$ est $CO_2R_8$, où $R_8$ est un hydrogène ou un alkyle, ou un autre ester de ceux-ci ou un sel de ceux-ci.

**11.** Un composé selon l'une quelconque des revendications précédentes pour l'utilisation dans le traitement d'une maladie d'un animal à sang chaud par inhibition de l'enzyme 5-lipoxygénase.

**12.** Une composition pharmaceutique qui comprend un composé selon l'une quelconque des revendications 1 à 11 et un véhicule ou excipient pharmaceutiquement acceptables.

**13.** Un procédé de production d'un composé selon l'une quelconque des revendications 1 à 11, qui comprend

a) pour préparer un composé de formule 1 dans laquelle $R_5$ est un hydrogène et G est -OH, la réaction d'un composé de formule 7

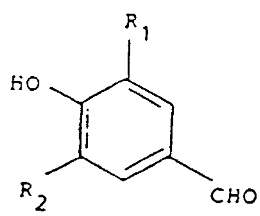

7

avec un acide aryl- ou hétéroarylacétique 8

$ArCH_2CO_2H$ 8

pour former un composé de formule 1" ayant la structure

1"

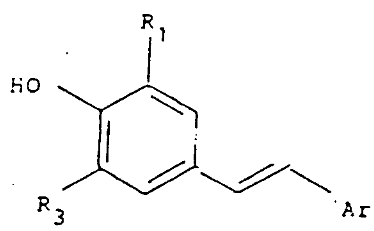

dans laquelle Ar, $R_1$ et $R_2$ sont définis comme ci-dessus,

b) pour préparer un composé de formule 1, dans laquelle $R_5$ est $CO_2R_8$ et G est -OH, la réaction d'un composé de formule 7, comme défini ci-dessus, avec un ester d'acide aryl- ou hétéroarylacétique de formule 9

$ArCH_2CO_2R_8$ 9

pour former un composé de formule 10

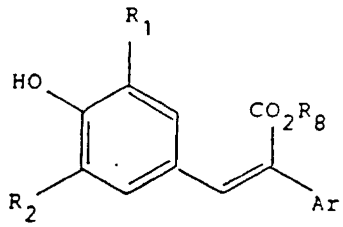

10

dans laquelle Ar, $R_1$ et $R_2$ sont définis comme ci-dessus et $R_8$ est un alkyle en $C_1$-$C_3$,

c) pour préparer un composé de formule 1 dans laquelle $R_8$ est un hydrogène, l'hydrolyse d'un ester de formule 10'

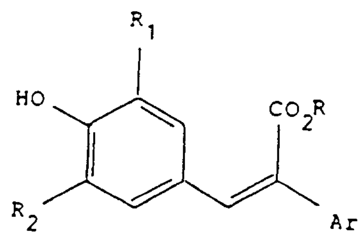

10'

dans laquelle R, qui peut être $R_8$ lorsque celui-ci est un alkyle en $C_1$-$C_3$, est un groupe formant un ester pouvant être éliminé de façon connue en soi, pour former l'acide libre 11

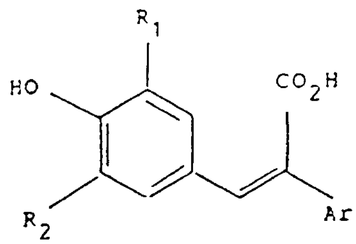

11

dans lequel Ar, $R_1$ et $R_2$ sont définis comme ci-dessus,

d) pour préparer un composé de formule 1 dans laquelle $R_5$ est un hydrogène, la décarboxylation d'un acide de formule 11 pour former un composé de formule 1",

e) pour préparer un composé de formule 1 dans laquelle $R_5$ est un alkyle en $C_1$-$C_5$, la déprotection d'un composé de formule 12

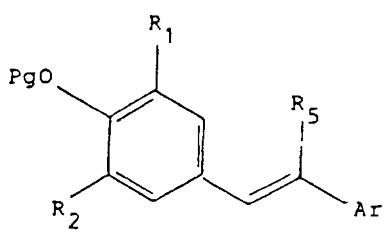

12

dans laquelle $R_1$, $R_2$ et Ar sont définis comme ci-dessus et Pg est un groupe hydroxy-protecteur

f) pour préparer un composé de formule 13

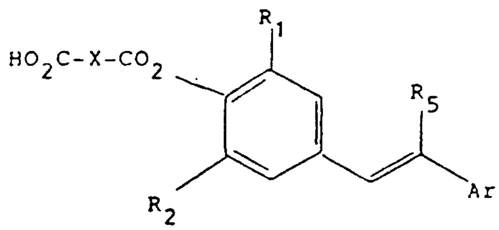

13

la réaction d'un composé de formule 1 dans laquelle G est OH avec un composé de formule

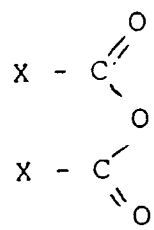

g) pour préparer un composé de formule 1 dans laquelle G est le groupe

$$(R_{10})(R_{11})N(\underset{R_{12}}{\underset{|}{C}H})_n-CO_2-$$

15

défini comme ci-dessus,
la réaction d'un composé de formule 1 dans laquelle G est OH et $R_5$ est un alkyle ou d'un composé de formule 10' défini comme ci-dessus, avec un composé de formule

$$(R_{10'})(R_{11'})N(\underset{R_{12}}{\underset{|}{C}H})_nCOOH$$

16

dans laquelle $R_1$, $R_2$ et $R_5$ sont définis comme ci-dessus, $R_{10'}$ et $R_{11'}$ sont un hydrogène ou un alkyle, sous réserve que lorsque l'un est un hydrogène l'autre est un alkyle ou un groupe protecteur éliminable et $R_{12}$ est un hydrogène, un alkyle ou un aryle qui peuvent contenir au moins un hétéroatome, et où $R_{11'}$ et $R_{12}$ peuvent ensemble former un cycle, et n est un entier de 1 à 5, puis l'élimination des groupes $R_{10'}$ et/ou $R_{11'}$ lorsqu'ils sont des groupes protecteurs, et, si cela est nécessaire ou souhaité, l'élimination du groupe R ;

et ensuite, si on le désire, le traitement du produit des procédés a) à g) selon une ou plusieurs des étapes de finition suivantes, dans un ordre désiré quelconque :

i) élimination d'un groupe ester $R_8$ pour obtenir un acide libre

ii) estérification d'un acide libre pour former un ester pharmaceutiquement acceptable dans lequel le groupe ester peut être clivé in vivo pour former l'acide libre

iii) formation d'un sel pharmaceutiquement acceptable de l'acide libre, par exemple le sel d'un métal alcalin ou d'un métal alcalino-terreux comme le sodium, le potassium ou le calcium

iv) la formation d'un sel d'addition d'acide d'un composé de formule 1, en particulier d'un tel composé dans lequel $R_3$ est le groupe

$$(R_{10})(R_{11})N\underset{\displaystyle R_{12}}{\underset{|}{(CH)}}_n-.$$

**14.** Utilisation d'un composé selon l'une quelconque des revendications 1 à 11 pour la préparation d'une composition pharmaceutique pour le traitement de l'inflammation et dans les maladies où des produits du métabolisme de la 5-lipoxygénase jouent un rôle, telles que l'asthme, le psoriasis, la polyarthrite rhumatoïde et les affections intestinales inflammatoires non spécifiques et pour inhiber l'enzyme cyclooxygénase.